(19) Europäisches Patentamt
European Patent Office
Office européen des brevets

(11) Numéro de publication : **0 418 143 B1**

# (12) FASCICULE DE BREVET EUROPEEN

(45) Date de publication du fascicule du brevet : **23.03.94 Bulletin 94/12**

(51) Int. Cl.⁵ : **C07D 211/60,** C07D 211/78, C07D 309/08, C07D 309/28, A61K 31/35, A61K 31/445

(21) Numéro de dépôt : **90402496.5**

(22) Date de dépôt : **11.09.90**

(54) **Dérivés dicarboxyliques renfermant un hétérocycle azoté ou oxygéné, leur procédé de préparation et leur application comme médicaments.**

(30) Priorité : **12.09.89 FR 8911879**

(43) Date de publication de la demande : **20.03.91 Bulletin 91/12**

(45) Mention de la délivrance du brevet : **23.03.94 Bulletin 94/12**

(84) Etats contractants désignés : **AT BE CH DE DK ES FR GB GR IT LI LU NL SE**

(56) Documents cités : **EP-A- 0 284 461**

(73) Titulaire : **ROUSSEL-UCLAF**
**35, Boulevard des Invalides**
**F-75007 Paris (FR)**

(72) Inventeur : **Agouridas, Constantin**
**67, Quai d'Orsay**
**F-75007 Paris (FR)**
Inventeur : **Fauveau, Patrick**
**40, Avenue Camille Desmoulins**
**F-93190 Levry-Gargan (FR)**

(74) Mandataire : **Tonnellier, Marie-José et al**
**111, route de Noisy B.P. no 9**
**F-93230 Romainville (FR)**

EP 0 418 143 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux dérivés dicarboxyliques renfermant un hétérocycle azoté ou oxygéné, leur procédé de préparation et leur application comme médicaments.

L'invention a pour objet les composés de formule (I) :

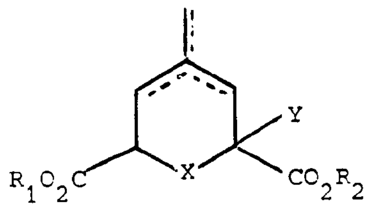

(I)

dans laquelle :

- les traits pointillés représentent une double liaison éventuelle endo ou exo ;
- $R_1$ et $R_2$ identiques ou différents représentent un atome d'hydrogène, un radical alkyle, alkényle, ou alkynyle renfermant jusqu'à 8 atomes de carbone,
  un radical aryle renfermant jusqu'à 14 atomes de carbone, un radical arylalkyle renfermant jusqu'à 18 atomes de carbone ou un radical

  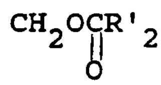

  dans lequel $R'_2$ représente un radical alkyle renfermant jusqu'à 8 atomes de carbone ou un radical aryle renfermant jusqu'à 14 atomes de carbone ;
- X représente un atome d'oxygène ou un radical NR, R représentant un atome d'hydrogène, un radical CHO, un radical $CO_2R'$, R' représentant un atome d'hydrogène ou un radical alkyle renfermant jusqu'à 8 atomes de carbone ;
- Y représente un atome d'hydrogène, un radical alkyle, alkényle ou alkynyle renfermant jusqu'à 8 atomes de carbone éventuellement substitué par un ou plusieurs atomes d'halogène ou un radical hydroxyle, à la condition que si Y représente un radical hydroxyle, X ne représente pas un radical NH ainsi que leurs sels d'addition avec les acides organiques ou minéraux ou avec les bases.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléïque, fumarique, succinique, tartrique, citrique, oxalique, glyoxylique, aspartique, alcanesulfoniques tels que les acides méthane ou éthanesulfoniques, arylsulfoniques, tels que les acides benzène ou paratoluènesulfoniques et arylcarboxyliques. Les sels des dérivés de l'invention peuvent être formés avec des bases organiques et minérales. Parmi les bases minérales, on peut citer les hydroxydes alcalins et alcalino-terreux, tels que par exemple, les hydroxydes de sodium, de potassium, de lithium et de calcium, l'hydroxyde de magnésium ou d'ammonium. Parmi les bases organiques, on peut citer les amines alkylées substituées ou non substituées, telles que par exemple, la triméthylamine, la méthylamine, la propylamine, la N,N-diméthyléthanolamine ou la tris(hydroxyméthyl) méthylamine ; on peut citer également des acides aminés basiques tels que, par exemple, la lysine ou l'arginine ; on peut citer encore d'autres bases telles que, par exemple, la glucosamine ou la procaïne.

Lorsque l'un des substituants représente un radical alkyle, il s'agit de préférence du radical éthyle, propyle, isopropyle ou butyle, ou d'un radical insaturé comme le radical vinyle, allyle, éthynyle ou propynyle.

Lorsque Y représente un radical alkyle substitué par un ou plusieurs atomes d'halogène, il s'agit de préférence d'un radical alkyle substitué par un ou plusieurs atomes de fluor ou de chlore, par exemple, les radicaux $-CHF_2$-, $-CH_2F$, $-CHCl_2$, $-CH_2Cl$.

Lorsque l'un des substituants représente un radical aryle, il s'agit de préférence du radical phényle.

Lorsque l'un des substituants représente un radical arylalkyle, il s'agit de préférence du radical benzyle.

Parmi les composés préférés de l'invention, on peut citer les composés dans lesquels les traits pointillés représentent une double liaison exo, ainsi que ceux dans lesquels les traits pointillés ne représentent pas une double liaison, ainsi que leurs sels d'addition avec les acides organiques ou minéraux.

Parmi les composés préférés de l'invention, on peut également citer, les composés dans lesquels $R_1$ et $R_2$ représentent un atome d'hydrogène ou encore ceux dans lesquels Y représente un radical hydroxyle, ainsi

que ceux dans lesquels Y représente un radical acétylénique, ainsi que leurs sels d'addition avec les acides organiques ou minéraux, ou avec les bases.

L'invention a plus particulièrement pour objet les composés dans lesquels Y représente un atome d'oxygène ainsi que ceux dans lesquels X représente un groupement $NCO_2R'$, R' étant défini comme précédemment, notamment les composés dans lesquels X représente un radical $NCO_2CH_3$ ainsi que leurs sels d'addition avec les acides organiques ou minéraux.

L'invention a plus particulièrement pour objet les produits dont la préparation est donnée ci-après dans la partie expérimentale, notamment les produits des exemples 1, 2, 22.

Les dérivés, objet de la présente invention, possèdent de très intéressantes propriétés pharmacologiques, ils sont doués notamment d'intéressantes propriétés anti-bactériennes et immunologiques.

Ces propriétés sont illustrées plus loin dans la partie expérimentale.

Ces propriétés justifient l'utilisation des produits de l'invention ainsi que de leurs sels, à titre de médicaments.

La présente demande a ainsi également pour objet l'application, à titre de médicaments, des dérivés de formule (I) ainsi que de leurs sels d'addition pharmaceutiquement acceptables.

Parmi les médicaments préférés, on peut citer les composés préférés cités ci-dessus et particulièrement ceux des exemples 1, 2 et 22.

Les médicaments selon l'invention trouvent leur emploi, par exemple, dans l'antibiothérapie vis-à-vis des germes bactériens, des champignons, des levures (candida albicans...) dans la thérapie anti-virale, et dans la chimiothérapie anti-cancéreuse, seuls ou en association, et enfin comme adjuvants à une antibiothérapie classique ou à la vaccination.

L'invention a enfin pour objet les compositions pharmaceutiques qui renferment au moins un dérivé précité ou l'un de ses sels d'addition avec les acides ou avec les bases pharmaceutiquement acceptables, à titre de principe actif.

A titre de médicaments, les dérivés répondant à la formule (I) et leurs sels d'addition avec les acides ou les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme, par exemple, les comprimés, simples ou dragéifiés, les gélules, les granulés, les suppositoires, les préparations injectables, elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants et les conservateurs.

L'invention a également pour objet un procédé de préparation des composés de formule (I) caractérisé en ce que l'on soumet un composé de formule (II) :

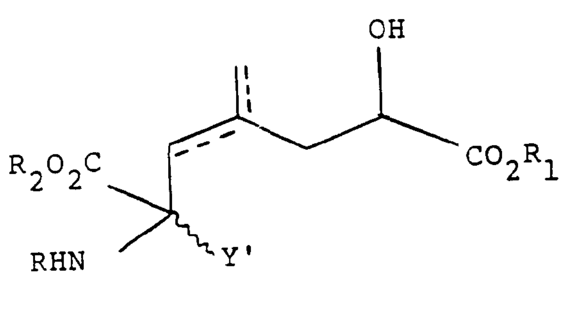

(II)

dans laquelle $R_1$ et $R_2$ conservent la même signification que précédemment, Y' représente soit les mêmes valeurs que Y, soit un précurseur de Y et R représente un radical CHO ou $CO_2$alc, alc représentant un radical alkyyle renfermant jusqu'à 8 atomes de carbone ;

ou bien à l'action d'un composé de formule (III) :

Hal $SO_2$alc (III)

dans laquelle Hal représente un atome d'halogène et alc représente un radical alkyle renfermant jusqu'à 8 atomes de carbone pour obtenir le composé de formule (IV) :

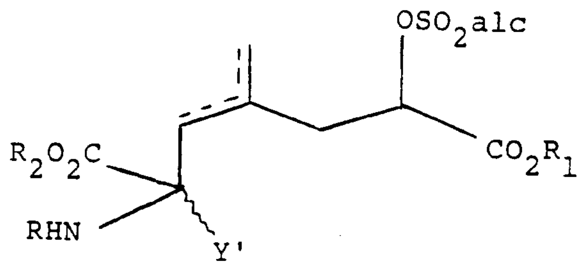

(IV)

que l'on soumet à l'action d'un agent favorisant la substitution nucléophile intramoléculaire pour obtenir le composé de formule ($I_A$) :

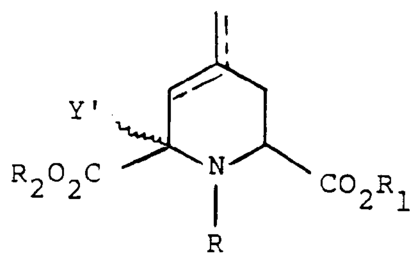

($I_A$)

<u>ou bien</u> dans le cas ou Y' représente un atome d'hydrogène et R un radical CHO, à l'action d'un agent capable de libérer la fonction aminée pour obtenir le composé de formule (V) :

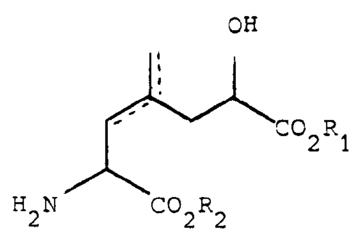

(V)

que l'on soumet à l'action du N-chlorosuccinimide ou du benzènesulfonate de 4-formyl 1-méthyl pyridinium, puis soumet le produit obtenu à l'action d'une base, puis soumet le produit obtenu à l'action d'un agent d'hydrolyse pour obtenir le composé de formule ($I_B$) :

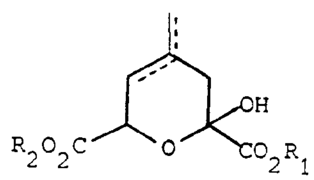

($I_B$)

<u>ou bien</u> dans le cas où Y représente un atome d'hydrogène et R un radical CHO à l'action d'un agent oxydant pour obtenir le composé de formule ($I_C$) :

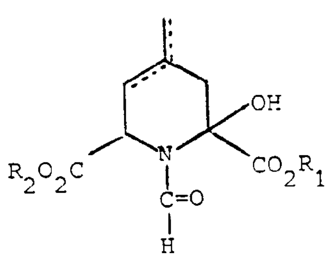

($I_C$)

puis soumet le cas échéant les composés de formule ($I_A$), ($I_B$) et ($I_C$) à la totalité ou à une partie seulement des opérations suivantes dans un ordre quelconque :

- réduction éventuelle de la double liaison endo ou exo ;
- traitement de Y' pour obtenir Y ;
- réduction totale ou sélective du substituant Y lorsque celui-ci est insaturé ;
- déprotection de la fonction aminé ;
- salification.

Dans un mode de réalisation préféré du procédé de l'invention,

- le dérivé de formule (III) utilisé est le chlorure de mesyle ou le chlorure de tosyle ;
- l'agent favorisant substitution nucléophile intramoléculaire est une base forte comme le carbonate de potassium ou un autre carbonate alcalin ;
- l'agent capable de libérer la fonction amine du composé de formule (II) est un acide fort comme par exemple l'acide chlorhydrique ;
- la base utilisée est le diazabicyclo undécène ou la triéthylamine ;
- l'agent d'hydrolyse est l'acide oxalique ;
- l'agent oxydant que l'on fait réagir sur le composé de formule (II) est le réactif de JONES $CrO_3$-$H_2SO_4$, $H_2O$ ;
- l'hydrolyse des fonctions esters est réalisée de préférence par saponification à l'aide d'une base minérale comme, la soude ou la potasse, suivie éventuellement d'une traitement par une résine acide ;
- le groupement protecteur du radical acétylénique est un groupement triméthylsilyl ou tout groupement connu de l'homme de métier ;
- l'agent de clivage du groupement triméthylsilyle éventuel est le fluorore de potassium ou le fluorore de tétrabutylammonium ou tout autre moyen connu de l'homme de métier ;
- le catalyseur d'hydrogénation est le palladium sur charbon actif empoisonné ou non par la quinoléine ;
- la déprotection de la fonction amine est réalisée par action d'un acide minéral tel que l'acide chlorhydrique, ou d'un acide organique tel que l'acide trifluoroacétique ;
- la salification est réalisée par addition d'acide ou de base au milieu réactionnel.

Les composés de formule (II) utilisés comme produits de départ peuvent être préparés selon le procédé décrit dans les brevets européens publiés sous les numéros 0 284 461 et 0 315 519 et dans la demande BF 89 05108. Certains composés de départ n'ont pas été décrits à ce jour, leur préparation est donnée ci-après dans la partie expérimentale.

L'invention a plus particulièrement pour objet un procédé caractérisé en ce que l'on soumet un composé de formule ($II_A$) :

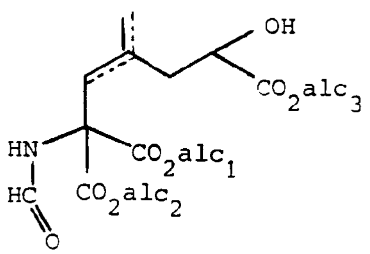

($II_A$)

dans laquelle $alc_1$, $alc_2$ et $alc_3$ identiques ou différents représentent un radical alkyle renfermant jusqu 'à 8 atomes de carbone à l'action du chlorure de mésyle pour obtenir le composé de formule ($IV_A$) :

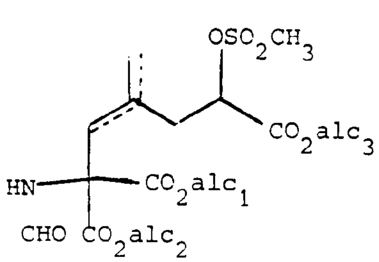

($IV_A$)

que l'on soumet à l'action d'un agent de cyclisation pour obtenir le composé de formule ($I'_A$) :

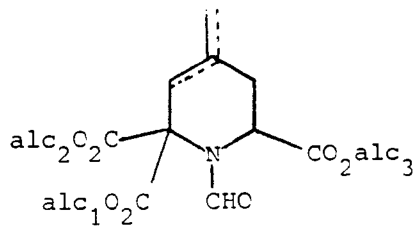

(I'$_A$)

que l'on soumet à l'action d'un agent de décarboxylation pour obtenir le composé de formule (I"$_A$) :

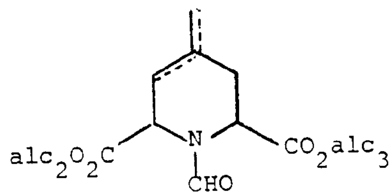

(I"$_A$)

Dans un mode de réalisation préféré, la décarboxylation peut être effectuée suivant la technique de Krapcho.

L'invention a également pour objet préféré un procédé caractérisé en ce que l'on soumet un composé de formule (II$_B$) :

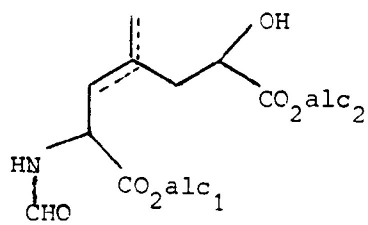

(II$_B$)

dans laquelle $alc_1$ et $alc_2$ identiques ou différents représentent un radical alkyle renfermant jusqu'à 8 atomes de carbone, à l'action d'un agent d'oxydation pour obtenir le composé de formule (I'$_C$) :

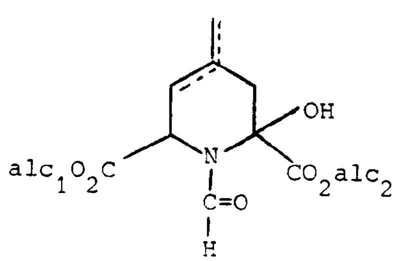

(I'$_C$)

L'invention a en outre comme objet préféré un procédé caractérisé en ce que l'on soumet un composé de formule (II$_C$) :

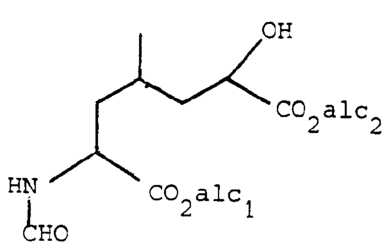

(II$_C$)

dans laquelle $alc_1$ et $alc_2$ identiques ou différents représentent un radical alkyle renfermant jusqu'à 8 atomes de carbone à l'action d'un agent de déprotection de la fonction amine, pour obtenir le composé de formule ($II_{B'}$) :

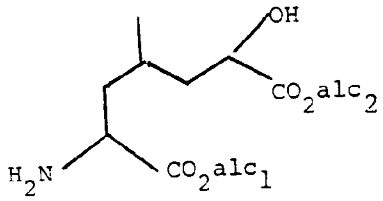

($II_{B'}$)

que l'on soumet à l'action du N-chlorosuccinimide ou du benzènesulfonate de 4-formyl 1-méthyl pyridinium, puis à l'action d'une base, puis à l'action d'un agent d'hydrolyse pour obtenir le composé de formule ($I'_B$) :

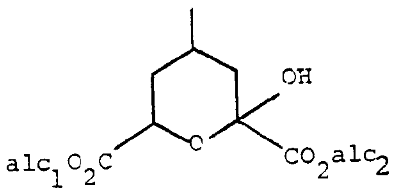

($I'_B$)

Les exemples suivants illustrent l'invention sans toutefois la limiter.

**Exemple 1: 2-éthynyl 4-méthylène 1,2,6-pipéridinetricarboxylate de 1-méthyle (isomère A).**

STADE A : 4-méthylène 2-[2-(triméthylsilyl) éthynyl] 1,2,6-pipéridin-carboxylate de 1,2-diméthyl 6-éthyl (isomères A et B).

On ajoute 2,95 g de carbonate de potassium dans une solution renfermant 10 g de 6-(méthanesulfonyl) 2-[(méthoxycarbonyl) amino] 4-méthylène 2-[2-(triméthylsilyl) éthynyl] heptanedioate de 7-éthyl 1-méthyle obtenu comme décrit dans la préparation 1 dans le BF. 89 05108 et 90 $cm^3$ de diméthylformamide. On maintient le mélange réactionnel sous atmosphère d'argon à 90°C pendant 3 heures. On dilue à l'éther le produit obtenu, filtre les sels minéraux et amène à sec. On chromatographie le résidu sur silice en éluant par le mélange cyclohexane-acétate d'éthyle (8-2). On obtient ainsi 2,45 g d'isomère A brut et 2,72 g d'isomère B pur. Après une nouvelle chromatographie sur silice, en éluant par le mélange cyclohexane-acétate d'éthyle (8-2), on obtient 1,53 g d'isomère A pur.
Eluant : cylohexane, acétate d'éthyle (8,2).
Isomère A : rf=0,26 et Isomère B : rf=0,2.

STADE B : 2-éthynyl 4-méthylène 1,2,6-pipéridinetricarboxylate de 1,2-diméthyl 6-éthyle (isomère A).

On ajoute 0,465 g de fluorure de potassium dans une solution renfermant 1,53 g de produit préparé au stade précédent (isomère A) dans 15 $cm^3$ de diméthylformamide. On maintient le mélange réactionnel sous agitation pendant 2 heures. On dilue à l'éther, filtre les sels minéraux, lave la phase organique à l'eau salée, sèche et évapore à sec. On obtient ainsi 1,31 g de produit que l'on chromatographie sur silice en éluant par le cyclohexane puis par le mélange cyclohexane-acétate d'éthyle (8-2). On obtient ainsi 1,016 g de produit recherché rf=0,2.

STADE C : 2-éthynyl 4-méthylène 1,2,6-pipéridinetricarboxylate de 1-méthyle (isomère A).

On ajoute 0,41 $cm^3$ de soude 2N dans une solution renfermant 103 mg de produit préparé au stade précédent dans 1 $cm^3$ d'éthanol. On agite le mélange réactionnel pendant 24 heures à la température ambiante, ajoute 0,1 $cm^3$ de soude 2N et poursuit l'agitation pendant 24 heures. On concentre, amène à pH 6 et purifie sur résine Dowex 50 W x 8. On obtient ainsi 28 mg de produit recherché. rf=0,4 (éluant : chlorure de méthylène, méthanol, acétate acétique 80-20-5).

**Exemple 2: 2-éthynyl 4-méthylène 1,2,6-pipéridinetricarboxylate de 1-méthyle (isomère B).**

STADE A : 2-éthynyl 4-méthylène 1,2,6-pipéridinetricarboxylate de 1,2-diméthyl 6-éthyle.

En opérant comme au stade B de l'exemple 1 à partir du 4-méthylène 2-[2-(triméthylsilyl) éthynyl] 1,2,6-pipéridine tricarboxylate de 1,2-diméthyl 6-éthyle (isomère B), on obtient 1,48 g de produit recherché. rf=0,15 (cyclohexane-acétate d'éthyle (8-2)).

STADE B : 2-éthynyl 4-méthylène 1,2,6-pipéridinetricarboxylate de 1-méthyle (isomère B).

On ajoute à 0°C 4,9 $cm^3$ de soude 2N dans une solution renfermant 1,39 g de produit préparé au stade précédent et 14 $cm^3$ d'éthanol. On agite la solution réactionnelle pendant 20 heures à la température ambiante. On ajoute 1 $cm^3$ de lessive de soude et agite à la température ambiante pendant 4 heures. On dilue à l'eau, ajoute de la résine Dowex 50 W x 8 jusqu'à pH 1, filtre, rince à l'eau, à l'éthanol et évapore à sec. On obtient 1,21 g de produit recherché que l'on chromatographie sur silice en éluant par le mélange éthanol-ammoniaque (8-2). On obtient 610 mg de produit recherché rf=0,2.

**Exemple 3: 2-éthényl 4-méthylène pipéridine 1,2,6-tricarboxylate de 1-méthyle.**

STADE A : 2-éthényl 4-méthylène 1,2,6-pipéridinetricarboxylate de 1,2-diméthyl-6-éthyle.

On dissout à la température ambiante 3,57 g de produit obtenu au stade B de l'exemple 1 dans un mélange de 350 $cm^3$ d'éthanol et 0,35 $cm^3$ de quinoléine. On ajoute 3,5 g de palladium à 5% sur sulfate de baryum. On hydrogène sous 1300 mm de mercure. On filtre, rince à l'éthanol et amène à sec. On obtient 3,9 g de produit recherché brut que l'on purifie par chromatographies successives sur silice en éluant par le mélange cyclohexane-acétate d'éthyle (8-2), puis par le mélange chlorure de méthylène, acétate d'éthyle (95-5), puis par le mélange cyclohexane-acétate d'éthyle (8-2). On obtient ainsi 1,58 g de produit recherché. rf=0,15 cyclohexane-acétate d'éthyle (8-2).

STADE B : 2-éthényl 4-méthylène pipéridine 1,2,6-tricarboxylate de 1-méthyle (isomère A).

On dissout 450 mg de produit préparé au stade précédent dans 2,5 $cm^3$ d'éthanol. On glace et ajoute 2,5 $cm^3$ de lessive de soude. On agite à la température ambiante pendant 24 heures. On dilue à l'eau, glace, ajoute de la résine Dowex 50 W x 8, filtre, rince à l'eau, amène à sec et lyophilyse après avoir filtré la solution obtenue. On obtient ainsi 306 mg de produit brut que l'on chromatographie sur silice en éluant par le mélange éthanol-ammoniaque (8-2). On obtient ainsi 228 mg de produit recherché. rf=0,3.

**Exemple 4: 2-éthényl 4-méthylène pipéridine 1,2,6-tricarboxylate de 1-méthyle (isomère B).**

STADE A : 2-éthényl 4-méthylène 1,2,6-pipéridinetricarboxylate de 1,2-diméthyl-6-éthyle.

On dissout 1,55 g de produit préparé au stade A de l'exemple 2 dans 150 $cm^3$ d'éthanol. On ajoute à cette solution 0,16 $cm^3$ de quinoléine et 1,49 g de palladium à 5 % sur sulfate de baryum. On hydrogène la suspension obtenue jusqu'à absorption du volume théorique. On filtre, rince à l'éthanol et évapore à sec. On obtient 1,6 g de produit que l'on chromatographie sur silice en éluant par le mélange cyclohexane acétate-d'éthyle (8-2). On obtient ainsi 1,08 g de produit recherché rf=0,15.

STADE B : 2-éthényl 4-méthylène pipéridine-1,2,6-tricarboxylate de 1-méthyle.

On dissout 936 mg de produit préparé au stade précédent dans 10 volumes d'éthanol. On glace et introduit 10 volumes de lessive de soude. On agite pendant 48 heures. On dilue à l'eau, ajoute de la résine Dowex 50 W x 8 jusqu'à pH 2, agite pendant 2 heures à la température ambiante, rince à l'eau, concentre et lyophylise. On obtient 830 mg de produit que l'on purifie par chromatographie sur silice en éluant par le mélange éthanol ammoniaque (8-2) puis par l'eau. On lyophylise et obtient ainsi 400 mg de produit recherché rf=0,2.

**Exemple 5: Acide 1-formyl 4-méthylène 2,6-pipéridinedicarboxylique (isomère A).**

STADE A : 1-(formylamino) 5-méthanesulfonyloxy 3-méthylène 1,1,5-pentane tricarboxylate de triéthyle.

En opérant comme à l'exemple 1 stade A, à partir du 1-(formylamino) 5-hydroxy 3-méthylène 1,1,5-pentane tricarboxylate de triéthyle (préparé comme indiqué dans le brevet français 2611721 préparation B), on obtient le produit recherché.

STADE B : 1-formyl 4-méthylène 2,2,6-pipéridinetricarboxylate de triéthyle.

En opérant comme à l'exemple 1 stade B, à partir de 10 g du composé préparé au stade précédent, on obtient 6,95 g de produit recherché rf=0,3, éluant : cyclohexane-acétate d'éthyle (7-3).

STADE C : 1-formyl 4-méthylène 2,6-pipéridinedicarboxylate de diéthyle.

On agite pendant 3 heures à 165°C un mélange renfermant 5 g de produit préparé au stade précédent, 0,8 g de chlorure de sodium, 0,26 g d'eau et 15 $cm^3$ de diméthylsulfoxyde. On amène à la température ambiante, filtre, lave et évapore à sec. On obtient 4 g de produit que l'on purifie sur silice en éluant par le mélange cyclohexane-acétate d'éthyle (9-1), puis par le mélange cyclohexane-acétate d'éthyle (7-3). On obtient ainsi 3,1 g de produit recherché rf=0,5.

STADE D : Acide 1-formyl 4-méthylène 2,6-pipéridinecarboxylique (isomère A).

En opérant comme à l'exemple 1 stade C à partir de 997 mg du produit obtenu au stade précédent, on obtient 404 mg de produit recherché rf=0,2 éluant éthanol ammoniaque (8-2).

**Exemple 6: cis (±) trans 4-méthylène 2,6-pipéridinedicarboxylate de diéthyle chlorhydrate.**

STADE A : 4-méthylène 2,6-pipéridinedicarboxylate de diéthyle (isomère cis et isomère trans).

On ajoute 1,5 $cm^3$ d'acide chlorhydrique 12 N dans une solution renfermant 2,8 g de produit préparé au stade C de l'exemple 5 et 30 $cm^3$ d'éthanol. On porte au reflux pendant 2 heures le mélange réactionnel. On amène à sec, reprend à l'acétate d'éthyle, lave avec de l'hydrogénocarbonate à 10 %. On sèche la phase organique, filtre et amène à sec. On obtient 2,0 g de produit que l'on purifie sur silice en éluant par le mélange cyclohexane-acétate d' éthyle (8-2). On obtient 0,730 g de produit cis et 0,980 g de produit trans.

STADE B : cis (±) 4-méthylène 2,6-pipéridinedicarboxylate de diéthyle chlorhydrate.

On ajoute 2 $cm^3$ d'une solution d'éther chlorhydrique 2N dans une solution renfermant 0,2 g de produit isomère cis préparé au stade précédent dans 5 $cm^3$ d'éther éthylique. On amène à sec, reprend dans de l'éther isopropylique, filtre et rince à l'éther isopropylique. On obtient 0,205 g de produit recherché. F = 166°C.

**Exemple 7 : cis (±) 4-méthylène 2,6-pipéridinedicarboxylate de diéthyle chlorhydrate.**

En opérant comme au stade B de l'exemple 6 à partir de la base isomère trans préparée à l'exemple 6, on a obtenu 0,190 g de sel recherché isomère trans. F=109°C.

**Exemple 8: (±) trans acide 4-méthylène 2,6-pipéridinedicarboxylique.**

On ajoute 4,3 $cm^3$ d'une solution de soude 1N dans une solution renfermant 10 $cm^3$ d'éthanol et 0,5 g de produit trans préparé au stade A de l'exemple 6. On maintient le mélange réactionnel sous agitation pendant une heure. Après les traitements appropriés, on obtient 0,230 g de produit recherché F > 250°C.

**Exemple 9: (±) cis acide 4-méthylène 2,6-pipéridinedicarboxylique.**

On ajoute 3,5 $cm^3$ de solution de soude 1N dans une solution renfermant 0,4 g de produit cis préparé au stade A de l'exemple 6 et 10 $cm^3$ d'éthanol. On obtient après les traitements appropriés 0,240 g de produit recherché F > 250°C.

**Exemple 10: trans (±) 4-méthylène 1,2,6-pipéridinetricarboxylate de 1-méthyle.**

STADE A : 4-méthylène 1,2,6-pipéridinetricarboxylate de 2,6-diéthyl 1-méthyle (cis ±).

On ajoute à 0°C 0,346 $cm^3$ de triéthylamine dans une solution de 6 $cm^3$ de chlorure de méthylène renfermant 595 mg de produit trans préparé à l'exemple 6 stade A et 1,76 $cm^3$ de chloroformate de méthyle. On agite à la température ambiante pendant 3 heures. On dilue au chlorure de méthylène, lave à l'eau salée, sèche et évapore à sec, on obtient 680 mg de produit que l'on chromatographie sur silice en éluant par le mélange cyclohexane acétate d'éthyle (75-25) Rf=0,3.

STADE B : trans (±) 4-méthylène 1,2,6-pipéridinetricarboxylate de 1-méthyle.

On introduit 1,45 $cm^3$ de soude 2N à 0°C dans une solution renfermant 391 mg de produit préparé au stade précédent et 5 $cm^3$ d'éthanol. On agite 16 heures à la température ambiante. On dilue à l'eau et ajoute de la résine Dowex x 8 jusqu'à l'obtention d'un pH égal à 2. On obtient après les traitements appropriés 157 mg de produit recherché, rf=0,25 (éthanol-ammoniaque 8-2).

**Exemple 11: (cis ±) 4-méthylène 1,2,6-pipéridinetricarboxylate de 1-méthyle.**

STADE A : 4-méthylène 1,2-pipéridinetricarboxylate de 2,6-diéthyl 1-méthyle (cis ±).

On opère comme au stade de l'exemple 10 à partir de 595 mg de produit cis obtenu à l'exemple 6. On obtient après chromatographie sur silice (éluant : cyclohexane-acétate d'éthyle 9-1), 480 mg de produit recherché (rf=0,25 cyclohexane-acétone 8-2)).

STADE B : (cis ±) 4-méthylène 1,2,6-pipéridinetricarboxylate de 1- méthyle.

En opérant comme au stade B de l'exemple 10 à partir de 432 mg du produit obtenu au stade précédent, on obtient 132 mg de produit recherché rf=0,1 chlorure de méthylène-méthanol-acide acétique (95-5-5).

**Exemple 12: Acide 2-(difluorométhyl) 1-formyl 4-méthylène 2,6-pypéridine carboxylique.**

STADE A : 2-difluorométhyl 1-formyl 4-méthylène pipéridine 2,6-dicarboxylate d'éthyle.

On ajoute 0,39 g de carbonate de potassium dans une solution renfermant 1 g de 2-(difluorométhyl) 2-(formylamino) 4-méthylène 6-(méthylsulfonyl) heptanedioate de diéthyle préparé comme indiqué à l'exemple 12 stade F du brevet européen 0 315 519 dans 20 $cm^3$ de diméthylformamide. On agite la suspension ainsi obtenue pendant 1 h 30 à 100°C. On dilue à l'éther éthylique et filtre. On évapore à sec. On obtient 760 mg de produit recherché.

STADE B : Acide 2-(difluorométhyl) 1-formyl 4-méthylène 2,6-pipéridine carboxylique.

On ajoute 0,68 $cm^3$ de soude 1N dans une solution renfermant 54 mg de produit préparé au stade précédent dans 4 $cm^3$ d'éthanol. On agite à température ambiante pendant 60 heures. On ajoute 4 $cm^3$ d'eau et neutralise à pH environ 6 avec de la résine Amberlyst 15 $^R$ . On filtre et amène à sec. On reprend dans 8 $cm^3$ d'eau et on lyophilise. On obtient 47 mg de produit recherché sous forme de sel de sodium.

**Exemple 13: (±) 2-difluorométhyl 4-méthylène 2,6-pipéridinedicarboxylate.**

On dissout 760 mg de produit obtenu au stade A de l'exemple 12 dans 25 $cm^3$ d'éthanol. On ajoute 4 $cm^3$ d'acide chlorhydrique 12 N. On agite le mélange réactionnel ainsi obtenu pendant 2 h 30 au reflux. On ajoute 5 $cm^3$ d'eau et neutralise avec du carbonate acide de sodium. On amène à sec, reprend dans l'eau et extrait 3 fois à l'acétate d'éthyle. On sèche et évapore à sec. On obtient 520 mg de produit que l'on purifie par 2 chromatographies sur silice en éluant par le mélange cyclohexane-acétate d'éthyle (9-1). On obtient 225 mg de l'isomère A et 185 mg de l'isomère 8. (isomère A rf=0,55, isomère B rf=0,50 dans cyclohexane-acétate d' éthyle (6-4)).

**Exemple 14: (±) Acide 2-difluorométhyl 4-méthylène 2,6-pipéridine dicarboxylique (isomère A).**

On ajoute 1,2 $cm^3$ de soude 2N dans une solution renfermant 200 mg de produit obtenu à l'exemple 13 (isomère A) dans 7 $cm^3$ d'éthanol. On agite le mélange réactionnel ainsi obtenu pendant 32 heures à la température ambiante. On amène à pH environ 5 avec de l'acide chlorhydrique 2N. On évapore à sec. On reprend à l'eau. On chromatographie sur silice Dowex. On élue à l'eau, à l'ammoniaque 0,7 N puis à l'eau. On amène à sec et obtient 150 mg du produit brut que l'on reprend dans 20 $cm^3$ d'eau, filtre et lyophilise. On obtient 140 mg de produit recherché rf=0,4 éluant : butanol-acide acétique-eau (4-2-2).

**Exemple 15: (±) Acide 2-difluorométhyl 4-méthylène 2,6-pipéridine dicarboxylique (isomère B).**

On ajoute 1,3 $cm^3$ de soude 2N dans une solution renfermant 190 mg de produit obtenu à l'exemple 13 (isomère B) dans 7 $cm^3$ d'éthanol. On agite à la température ambiante pendant 30 heures. On neutralise à pH environ 5 avec de l'acide chlorhydrique 2N. On évapore à sec, reprend dans 5 cm3 d'eau et chromatographie sur résine Dowex en éluant à l'eau. On amène à sec, obtient 140 mg de produit recherché. On reprend dans 200 $cm^3$ d'eau, filtre et lyophilise. On obtient 130 mg de produit recherché, rf=04 butanol-acide acétique-eau (4-2-2).

**Exemple 16: 2-méthyl 4-méthylène 1,2,6-pipéridinetricarboxylate de 1-méthyle.**

STADE A : 2-[(méthoxycarbonyl) amino] 2-méthyl 4-méthylène 6-[(méthylsulfonyl) oxy] heptanedioate d'éthyle.

On dissout 3,6 g de 2-[(méthoxy carbonyl) amino] 2-méthyl 4-méthylène 6-[(méthylsulfonyl) oxy] heptane dioate d'éthyle préparé comme indiqué à l'exemple 11 Stade E du brevet européen 0 315 519 dans 80 $cm^3$ de diméthylformamide. On ajoute 1,46 g de carbonate de potassium. On agite vigoureusement la suspension obtenue pendant 4 heures à 100°C, dilue avec de l'éther éthylique, filtre et évapore à sec. On obtient 3,7 g de produit recherché brut que l'on purifie par chromatographie sur silice en éluant par le mélange cyclohexane-acétate d'éthyle-chlorure de méthylène (5-2-3). On obtient 1,05 g de produit isomère A pur et 0,56 g d'un mélange que l'on chromatographie en éluant avec le mélange cyclohexane-acétate d'éthyle-chlorure de méthylène (5-2-3). On obtient 300 mg d'isomère B et 50 mg d'isomère A.

STADE B : 2-méthyl 4-méthylène 1,2,6-pipéridinetricarboxylate de 1-méthyle (isomère A).

On ajoute 11,5 $cm^3$ de soude 6N dans une solution renfermant 290 mg de produit obtenu au stade précédent dans 2 $cm^3$ d'éthanol. On agite à la température ambiante pendant 4 jours. On amène à pH 3 avec de la résine Amberlyst 15 $^R$ , rince à l'eau, filtre et amène à sec. On obtient 255 mg de produit brut que l'on chromatographie sur silice en éluant par le mélange éthanol-ammoniaque 95/5 - 90/10 - 80/20. On reprend dans l'eau, filtre et lyophilise. On obtient 95 mg de produit recherché rf=0,25 éthanol-ammoniaque (9-1).

**Exemple 17: 2-méthyl 4-méthylène 1,2,6-pipéridinetricarboxylate de 1-méthyle.**

On ajoute 10 $cm^3$ de soude 6N dans une solution de 190 mg du produit préparé au stade A de l'exemple 16 (isomère B) dans 2 $cm^3$ d'éthanol. On agite 6 jours à la température ambiante. On neutralise le produit obtenu avec de la résine Dowex. On rince à l'eau jusqu'à pH 3, filtre et évapore à sec. On obtient 122 mg de produit que l'on purifie par chromatographie sur silice en éluant par le mélange méthanol-ammoniaque (80-20). On évapore à sec, reprend dans 20 $cm^3$ d'eau, filtre et lyophilise. On obtient 62 mg de produit recherché, rf=0,2 dans éthanol-ammoniaque (9-1).

**Exemple 18: 1-formyl 2-hydroxy 4-méthyl 2,6-pipéridinedicarboxylique de diéthyle.**

On ajoute 3,5 $cm^3$ de réactif de Jones [270 g de $CrO_3$, 400 $cm^3$ et 230 $cm^3$ de $H_2SO_4$ + quantité suffisante d'eau pour faire 1 litre], dans une solution renfermant 3 g de produit préparé comme indiqué ci-dessous et 60 $cm^3$ d'acétone. On laisse sous agitation pendant 1 h 30. On détruit l'excès d'oxydant par addition d'isopropanol. On ajoute de l'eau, neutralise par addition de carbonate de potassium en poudre, extrait au chlorure de méthylène, sèche sur sulfate de magnésium, filtre et amène à sec sous pression réduite. On obtient 3,2 g de produit que l'on chromatographie sur silice en éluant par le mélange cyclohexane-acétate d'éthyle (7-3). On obtient 970 mg du produit recherché rf=0,20.

**Préparation : 2-(formylamino) 6-hydroxy 4-méthyl heptanedioate de diéthyle.**

STADE A : 2-(formylamino) 6-hydroxy 4-méthylène heptanedioate de diéthyle.

On maintient sous agitation pendant 3 heures à 95°C, une solution renfermant 17,9 g de 1-(formylamino) 5-hydroxy 3-méthylène, 1,1,5-pentane tricarboxylate de triéthyle préparé comme indiqué dans la demande de brevet français 2 611 721, 300 $cm^3$ de diméthylformamide, 4,87 g de carbonate de césium et 13,15 g de 4 aminothiophénol. On évapore sous pression réduite la majeure partie du diméthylformamide, ajoute 100 $cm^3$ d'eau glacée, extrait au chlorure de méthylène, lave à l'acide chlorhydrique 2N, puis à l'eau salée. On sèche, filtre et amène à sec sous pression réduite. On obtient 15,2 g de produit que l'on chromatographie sur silice en éluant par le mélange chlorure de méthylène-méthanol (98-2). On obtient 11,06 g de produit recherché.

STADE B :2-(formylamino) 6-hydroxy 4-méthyl heptanedioate de diéthyle.

On laisse 15 heures sous une pression de 10 kg d'hydrogène, 5,74 g de produit obtenu au stade précédent, 150 $cm^3$ d'éthanol, 30 $cm_3$ d'acide acétique pur et 1,5 g de palladium à 10%. On filtre, rince à l'éthanol et amène à sec sous pression réduite. On reprend le résidu huileux dans du chlorure de méthylène, neutralise par une solution saturée de bicarbonate de sodium, décante, sèche et amène à sec. On obtient 5,4 g de produit recherché.

**Exemple 19: Acide 1-formyl 2-hydroxy 4-méthyl 2,6-pipéridinedicarboxylique (sel de sodium).**

On maintient pendant 15 heures sous agitation à la température ambiante un mélange de 143 mg de produit préparé à l'exemple 18, 2 $cm^3$ d'éthanol, 1 $cm^3$ de soude N. On obtient 155 mg de produit. On reprend à l'eau, filtre et lyophilise. On obtient 146 mg de produit recherché rf=0,35 butanol-acide acétique-alcool (4-2-2).

**Exemple 20: 1-formyl 2-hydroxy 4-méthyl 2,6-pipéridinedicarboxylate de sodium et de 6-éthyle.**

On ajoute 0,95 $cm^3$ de soude N dans une solution renfermant 287 mg de produit préparé à l'exemple 18 et 4 $cm^3$ d'éthanol. On laisse 4 heures sous agitation à la température ambiante puis amène à sec sous pression réduite. On ajoute 5 $cm^3$ d'eau et extrait plusieurs fois. On filtre la phase aqueuse et lyophilise. On obtient 258 mg de produit recherché rf=0,45 butanol-acide acétique-éthanol (4-2-2).

**Exemple 21: Acide 2-hydroxy 4-méthyl tétrahydro 2H-pyran-2,6-dicarboxylique (sel de sodium).**

STADE A : 2-amino 6-hydroxy 4-méthyl heptanedioate de diéthyle.

On maintient sous agitation à 90°C un mélange renfermant 2 g de 2-(formyl amino) 6-hydroxy 4-méthyl heptanedioate de diéthyle préparé comme indiqué précédemment, 20 $cm^3$ d'éthanol et 2 $cm^3$ d'acide chlorhydrique concentré. On refroidit, ajoute 10 $cm^3$ d'eau et neutralise par addition de carbonate de soude solide. On extrait au chlorure de méthylène, sèche, filtre et amène à sec. On obtient 1,7 g de produit recherché, rf=0,1 (éluant : chlorure de méthylène-acétate d'éthyle 1-1).

STADE B : 2-hydroxy 4-méthyl tétrahydro 2H-pyran 2,6-dicarboxylate de diéthyle (isomère A et isomère B).

On ajoute 840 mg de 4-formyl 1-méthyl pyridinium benzène sulfonate dans une solution renfermant 740 mg du produit préparé au stade A, 15 $cm^3$ de chlorure de méthylène et 5 $cm^3$ de diméthylformamide. On maintient le mélange réactionnel sous agitation pendant 45 minutes. On ajoute 1 $cm^3$ de diazabicyclo [5,4,0] undec-7-ène. On maintient à nouveau le mélange réactionnel sous agitation pendant 45 minutes. On refroidit à 5-10°C et ajoute 5 $cm^3$ d'une solution aqueuse saturée d'acide oxalique, maintient le mélange réactionnel sous agitation pendant 1 heure, extrait au chlorure de méthylène, lave avec une solution de bicarbonate de sodium, sèche, filtre et amène à sec. On obtient 630 mg de produit que l'on chromatographie sur silice en éluant par le mélange cyclohexane-acétate d'éthyle 9-1. On obtient 160 mg d'isomère A et 125 mg d'isomère B.

Isomère A :

Spectre RMN $CDCl_3$ 400 MHz

- 0,98 à 1,07 $C\underline{H}_3$-CH
- 1,22 à 1,40 m $C\underline{H}_3$-$CH_2$
- 1,4 à 3,05 m les $\underline{CH_2}$ et CH-$CH_3$

- 4,51 (dd) 2 types du

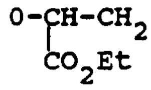

- 4,66 (dd)
- 4,6 à 4,40 (m) -C<u>H</u>$_2$-$CH_3$ de $CO_2CH_2CH_3$.

<u>Isomère B</u> :
Spectre RMN dans $CDCl_3$ 400 $MH_3$

- 0,99 (d) C<u>H</u>$_3$-CH
- 1,28 (t) 1,34 (t) C<u>H</u>$_3$-$CH_2$
- 2,00 (m)

- 1,71 (dd) J=13 et 11 Hz
- 1,75 (dd) J=13 et 4 Hz

} les $CH_2$ du cycle

- 2,14 (m) $CH_3$-CH-$CH_2$.

<u>STADE C</u> : Acide 2-hydroxy 4-méthyl tétrahydro 2H-pyran-2,6-dicarboxylique.

On maintient sous agitation pendant une nuit un mélange renfermant 111 mg d'un mélange d'isomères A et B comme préparés au stade précédent, 3 $cm^3$ d'éthanol et 0,85 $cm^3$ de soude N. On amène à sec sous pression réduite et ajoute quelques $cm^3$ d'eau distillée. On filtre et lyophilise. On obtient 91 mg de produit recherché.
RMN $D_20$ 300 $MH_3$
$CH_3$ 0,96 (d)
$CH_3$
C<u>H</u>$_2$, C<u>H</u> 1,0 à 2,3 ppm

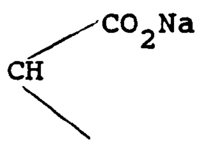

4,05 (dd)
4,27 (dd)
4,44 (dd) 4,51 (dd).
4,51 (dd)
1,35 (t J=13)
1,77 m
1,97 m

-CH $CO_2$ Na 4,08 4,27 dd:J=13.

**<u>Exemple 22</u>: 2-hydroxy-4-méthyl tétrahydro 2H-pyran-2,6-dicarboxylate de sodium (isomère B).**

En opérant comme au stade C de l'exemple précédent, à partir de l'isomère B obtenu au stade B de l'exemple 21, on obtient le produit recherché.
RMN $D_2O$ 300 $MH_3$
$CH_3$ en position 4 : 0,95 (d, J=6,5)
Hydrogènes du cycle en positions 3,4,5 : 1,15 (q, J=13), 1,35 (t, J=13), 1,77 (m), 1,97 (m),
Hydrogène du cycle en position 6 : 4,08, 4,27 (dd, J=13).

**<u>Exemple 23</u>: 2-hydroxy 4-méthyl tétrahydro 2H-pyran 2,6-dicarboxylate de sodium (isomère A).**

En opérant comme au stade C de l'exemple 21, à partir de l'isomère A obtenu au stade B de l'exemple

21, on obtient le produit recherché.
RMN $D_2O$ 300 $MH_3$
$CH_3$ en position 4 : 0,96 (d, J=6,5)
Hydrogènes du cycle en positions 3,4,5 : 1,21 (q, J=7), 1,25 à 3,0
Hydrogène du cycle en position 6 : 4,08, 4,27

**Exemple 24:**

On a préparé des comprimés répondant à la formule :

Produit de l'exemple 1 .................... 50 mg
Excipient q.s. pour un comprimé terminé à .. 250 mg

(détail de l'excipient : lactose, amidon, talc, stéarate de magnésium).

**Exemple 25:**

On a préparé des gélules répondant à la formule :
Produit de l'exemple 21............100 mg Excipient classique pour des gélules.

Activité antibactérienne (in vitro)

L'activité antibactérienne des produits revendiqués a été déterminée par la méthode de diffusion en milieu de Davis Mingioli additionné de 1 % d'agar. Les géloses utilisées sont coulées en boîtes de Petri à 48°C, après ensemencement à $5x10^{-5}$ germes/ml au moyen de la souche bactérienne test.

Les inocula proviennent d'une pré-culture de 24 heures en bouillon de Davis Mingioli. Après durcissement des géloses, les solutions aqueuses des produits étudiés sont introduites dans des puits (9 mm) creusés dans le milieu à l'aide d'un emporte-pièce. Les zones d'inhibitions observées (diamètre en mm) sont mesurées après incubation de 24 heures à 37°C.

| | Produit de l'exemple 1 (100 mg/l) | Produit de l'exemple 2 (100 mg/l) | Produit de l'exemple 21 (100 mg/l) |
|---|---|---|---|
| Escherichia coli 078 | 17,5 | 18 | |
| Salmonella typhimurium MZ11 | 31 | 34 | 19 |
| Enterobacter cloacae 1321E | 26 | 29 | |
| Providencia sp. DU48 | 29 | 28 | |

**Revendications**

**Revendications pour les Etats contractants suivants : AT, BE, CH, DE, DK, FR, GB, IT, LI, LU, NL, SE**

1. Les composés de formule (I) :

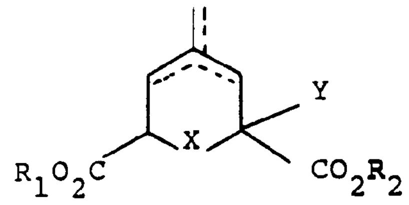

(I)

dans laquelle :

- les traits pointillés représentent une double liaison éventuelle endo ou exo ;
- $R_1$ et $R_2$ identiques ou différents représentent un atome d'hydrogène, un radical alkyle, alkényle, ou alkynyle renfermant jusqu'à 8 atomes de carbone,
  un radical aryle renfermant jusqu'à 14 atomes de carbone, un radical arylalkyle renfermant jusqu'à 18 atomes de carbone ou un radical

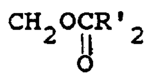

  dans lequel $R'_2$ représente un radical alkyle renfermant jusqu'à 8 atomes de carbone ou un radical aryle renfermant jusqu'à 14 atomes de carbone ;
- X représente un atome d'oxygène ou un radical NR, R représentant un atome d'hydrogène, un radical CHO, un radical $CO_2R'$, R' représentant un atome d'hydrogène ou un radical alkyle renfermant jusqu'à 8 atomes de carbone ;
- Y représente un atome d'hydrogène, un radical alkyle, alkényle ou alkynyle renfermant jusqu'à 8 atomes de carbone éventuellement substitué par un ou plusieurs atomes d'halogène ou un radical hydroxyle, à la condition que si Y représente un radical hydroxyle, X ne représente pas un radical NH ainsi que leurs sels d'addition avec les acides organiques ou minéraux ou avec les bases.

**2.** Les composés selon la revendication 1 dans lesquels les traits pointillés représentent une double liaison exo, ainsi que leurs sels d'addition avec les acides organiques ou minéraux ou avec les bases.

**3.** Les composés selon la revendication 1 dans lesquels les traits pointillés ne représentent pas une double liaison ainsi que leurs sels d'addition avec les acides organiques ou minéraux ou avec les bases.

**4.** Les composés selon la revendication 1, 2 ou 3 dans lesquels $R_1$ et $R_2$ représentent un atome d'hydrogène ainsi que leurs sels d'addition avec les acides organiques ou minéraux ou avec les bases.

**5.** Les composés selon l'une quelconque des revendications 1 à 4 dans lesquels Y représente un radical hydroxyle ainsi que leurs sels d'addition avec les acides organiques ou minéraux ou avec les bases.

**6.** Les composés selon l'une quelconque des revendications 1 à 4 dans lesquels Y représente un radical acétylénique ainsi que leurs sels d'addition avec les acides organiques ou minéraux ou avec les bases.

**7.** Les composés selon l'une quelconque des revendications 1 à 6 dans lesquels X représente un atome d'oxygène ainsi que leurs sels d'addition avec les acides organiques ou minéraux ou avec les bases.

**8.** Les composés selon l'une quelconque des revendications 1 à 6 dans lesquels X représente un groupement $NCO_2R'$, R' étant défini comme dans la revendication 1, ainsi que leurs sels d'addition avec les acides organiques ou minéraux ou avec les bases.

**9.** Les composés selon la revendication 7 dans lesquels X représente un radical $NCO_2CH_3$ ainsi que leurs sels d'addition avec les acides organiques ou avec les bases.

**10.** Les composés définis à la revendication 1 dont les noms suivent :

- l'acide 2-hydroxy-4-méthyl tétrahydro-2H-pyran-2,6-dicarboxylique ;
- le 2-éthynyl-4-méthylène 1,2,6-pipéridine tricarboxylate de 1-méthyle (isomère A et B) ainsi que leurs sels d'addition avec les acides organiques ou minéraux ou avec les bases.

**11.** A titre de médicaments, les composés de formule (I) définis à l'une quelconque des revendications 1 à 9 ainsi que leurs sels d'addition avec les acides organiques ou minéraux ou avec les bases pharmaceutiquement acceptables.

**12.** A titre de médicament, les composés de formule (I) définis à la revendication 10, ainsi que leurs sels d'addition avec les acides organiques ou minéraux ou avec les bases pharmaceutiquement acceptables.

**13.** Les compositions pharmaceutiques renfermant comme principe actif au moins un médicament défini à la revendication 11 ou 12.

**14.** Procédé de préparation des composés de formule (I) tels que définis à la revendication 1, caractérisé en ce que l'on soumet un composé de formule (II) :

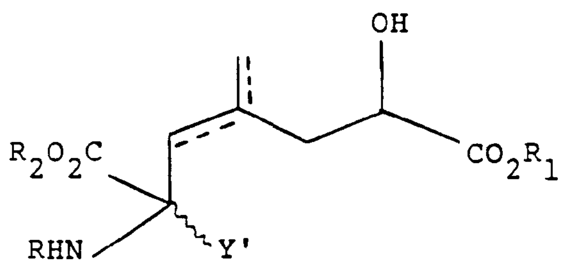

(II)

dans laquelle $R_1$ et $R_2$ conservent la même signification que précédemment, Y' représente soit les mêmes valeurs que Y, soit un précurseur de Y et R représente un radical CHO ou $CO_2$alc, alc représentant un radical alkyle renfermant jusqu'à 8 atomes de carbone ;
ou bien à l'action d'un composé de formule (III) :

Hal $SO_2$alc (III)

dans laquelle Hal représente un atome d'halogène et alc représente un radical alkyle renfermant jusqu'à 8 atomes de carbone pour obtenir le composé de formule (IV) :

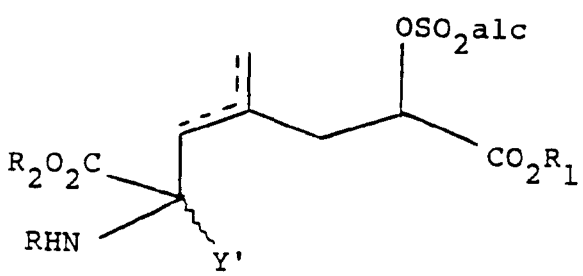

(IV)

que l'on soumet à l'action d'un agent favorisant la substitution nucléophile intramoléculaire pour obtenir le composé de formule ($I_A$) :

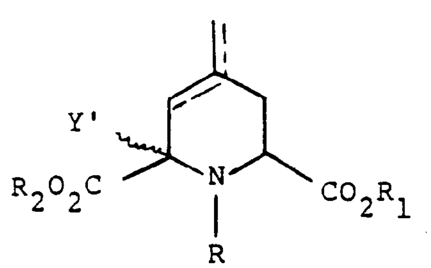

($I_A$)

ou bien dans le cas où Y' représente un atome d'hydrogène et R un radical CHO, à l'action d'un agent capable de libérer la fonction aminé pour obtenir le composé de formule (V) :

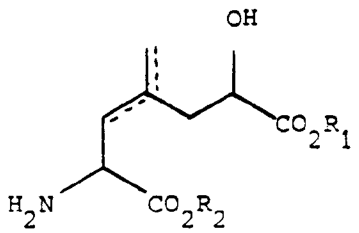

(V)

que l'on soumet à l'action du N-chlorosuccinimide ou du benzènesulfonate de 4-formyl 1-méthyl pyridinium, puis soumet le produit obtenu à l'action d'une base, puis soumet le produit obtenu à l'action d'un agent d'hydrolyse pour obtenir le composé de formule ($I_B$) :

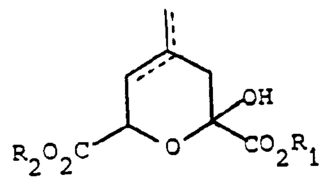

($I_B$)

ou bien dans le cas où Y représente un atome d'hydrogène et R un radical CHO à l'action d'un agent oxydant pour obtenir le composé de formule ($I_C$) :

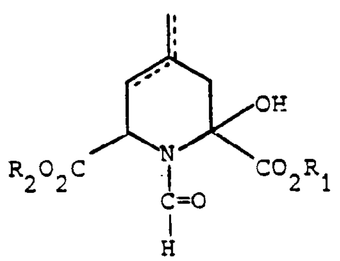

($I_C$)

puis soumet le cas échéant les composés de formule ($I_A$), ($I_B$) et ($I_C$) à la totalité ou à une partie seulement des opérations suivantes dans un ordre quelconque :

- réduction éventuelle de la double liaison endo ou exo ;
- traitement de Y' pour obtenir Y ;
- réduction totale ou sélective du substituant Y lorsque celui-ci est insaturé ;
- déprotection de la fonction aminé ;
- salification.

**15.** Procédé selon la revendication 14, caractérisé en ce que l'on soumet un composé de formule ($II_A$) :

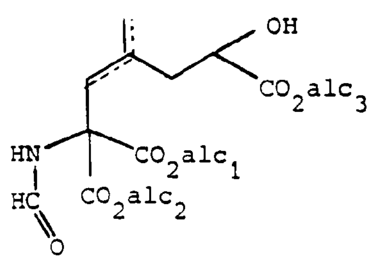

($II_A$)

dans laquelle $alc_1$, $alc_2$ et $alc_3$ identiques ou différents représentent un radical alkyle renfermant jusqu'à 8 atomes de carbone à l'action du chlorure de mésyle pour obtenir le composé de formule ($IV_A$) :

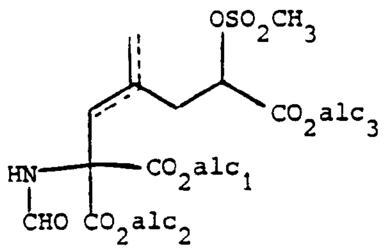

(IV$_A$)

que l'on soumet à l'action d'un agent de cyclisation pour obtenir le composé de formule (I'$_A$) :

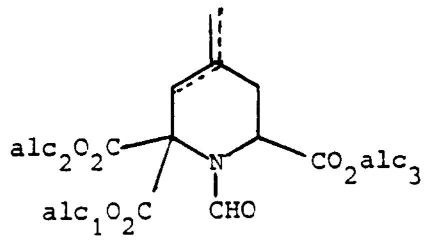

(I'$_A$)

que l'on soumet à l'action d'un agent de décarboxylation pour obtenir le composé de formule (I''$_A$):

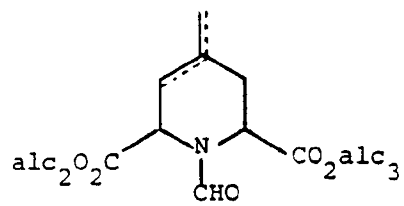

(I''$_A$)

**16.** Procédé selon la revendication 14, caractérisé en ce que l'on soumet un composé de formule (II$_B$) :

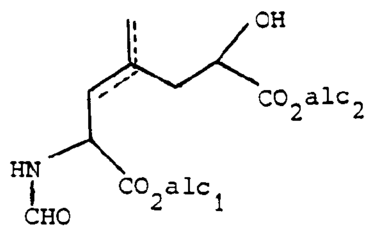

(II$_B$)

dans laquelle $alc_1$ et $alc_2$ identiques ou différents représentent un radical alkyle renfermant jusqu'à 8 atomes de carbone, à l'action d'un agent d'oxydation pour obtenir le composé de formule (I'$_C$) :

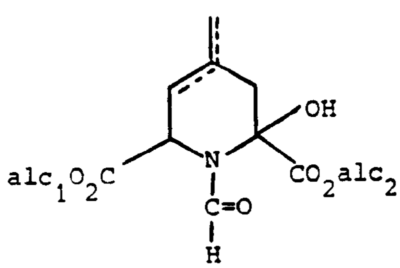

(I'$_C$)

**17.** Procédé de préparation selon la revendication 14, caractérisé en ce que l'on soumet un composé de formule (II$_C$) :

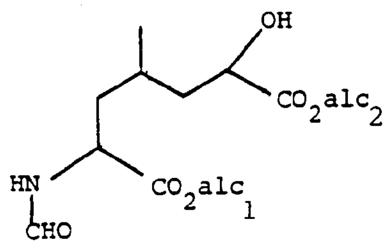

$(II_C)$

dans laquelle $alc_1$ et $alc_2$ identiques ou différents représentent un radical alkyle renfermant jusqu'à 8 atomes de carbone à l'action d'un agent de déprotection de la fonction amine, pour obtenir le composé de formule $(II_{B'})$ :

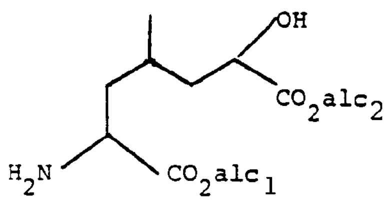

$(II_{B'})$

que l'on soumet à l'action du N-chlorosuccinimide ou du benzènesulfonate de 4-formyl 1-méthyl pyridinium, puis à l'action d'une base, puis à l'action d'un agent d'hydrolyse pour obtenir le composé de formule $(I'_B)$ :

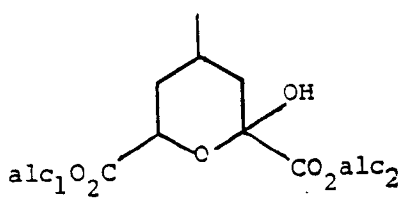

$(I'_B)$

**Revendications pour l'Etat contractant suivant : ES**

1. Procédé pour préparer les composés de formule (I) :

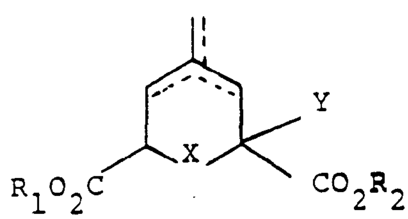

(I)

dans laquelle :

- les traits pointillés représentent une double liaison éventuelle endo ou exo ;
- $R_1$ et $R_2$ identiques ou différents représentent un atome d'hydrogène, un radical alkyle, alkényle, ou alkynyle renfermant jusqu'à 8 atomes de carbone,
  un radical aryle renfermant jusqu'à 14 atomes de carbone, un radical arylalkyle renfermant jusqu'à 18 atomes de carbone ou un radical

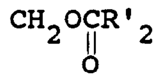

dans lequel $R'_2$ représente un radical alkyle renfermant jusqu'à 8 atomes de carbone ou un radical aryle renfermant jusqu'à 14 atomes de carbone ;

- X représente un atome d'oxygène ou un radical NR, R représentant un atome d'hydrogène, un radical CHO, un radical $CO_2R'$, R' représentant un atome d'hydrogène ou un radical alkyle renfermant jusqu'à 8 atomes de carbone ;
- Y représente un atome d'hydrogène, un radical alkyle, alkényle ou alkynyle renfermant jusqu'à 8 atomes de carbone éventuellement substitué par un ou plusieurs atomes d'halogène ou un radical hydroxyle, à la condition que si Y représente un radical hydroxyle, X ne représente pas un radical NH ainsi que leurs sels d'addition avec les acides organiques ou minéraux ou avec les bases, caractérisé en ce que l'on soumet un composé de formule (II) :

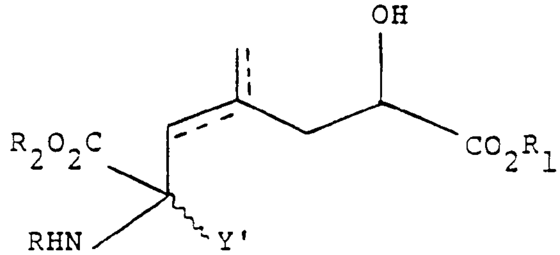

(II)

dans laquelle $R_1$ et $R_2$ conservent la même signification que précédemment, Y' représente soit les mêmes valeurs que Y, soit un précurseur de Y et R représente un radical CHO ou $CO_2$alc, alc représentant un radical alkyyle renfermant jusqu'à 8 atomes de carbone ;
ou bien à l'action d'un composé de formule (III) :

Hal $SO_2$alc (III)

dans laquelle Hal représente un atome d'halogène et alc représente un radical alkyle renfermant jusqu'à 8 atomes de carbone pour obtenir le composé de formule (IV) :

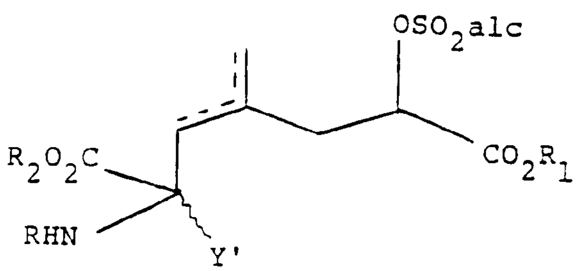

(IV)

que l'on soumet à l'action d'un agent favorisant la substitution nucléophile intramoléculaire pour obtenir le composé de formule ($I_A$) :

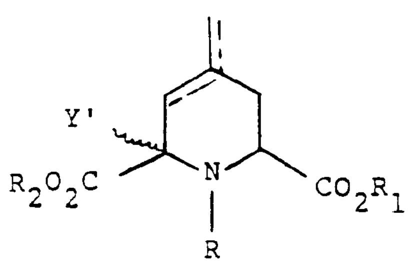

($I_A$)

ou bien dans le cas où Y' représente un atome d'hydrogène et R un radical CHO, à l'action d'un agent

capable de libérer la fonction aminé pour obtenir le composé de formule (V) :

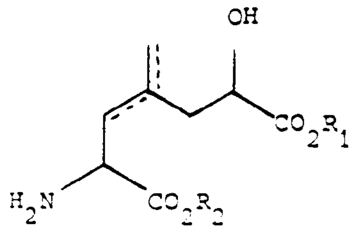

(V)

que l'on soumet à l'action du N-chlorosuccinimide ou du benzènesulfonate de 4-formyl 1-méthyl pyridinium, puis soumet le produit obtenu à l'action d'une base, puis soumet le produit obtenu à l'action d'un agent d'hydrolyse pour obtenir le composé de formule ($I_B$) :

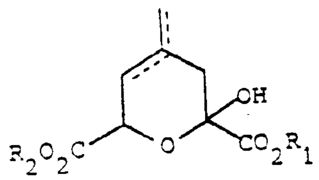

($I_B$)

ou bien dans le cas où Y représente un atome d'hydrogène et R un radical CHO à l'action d'un agent oxydant pour obtenir le composé de formule ($I_C$) :

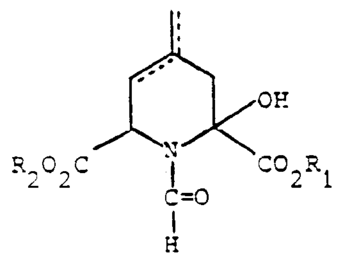

($I_C$)

puis soumet le cas échéant les composés de formule ($I_A$), ($I_B$) et ($I_C$) à la totalité ou à une partie seulement des opérations suivantes dans un ordre quelconque :

- réduction éventuelle de la double liaison endo ou exo ;
- traitement de Y' pour obtenir Y ;
- réduction totale ou sélective du substituant Y lorsque celui-ci est insaturé ;
- déprotection de la fonction aminé ;
- salification.

**2.** Procédé selon la revendication 1, caractérisé en ce que l'on soumet un composé de formule ($II_A$) :

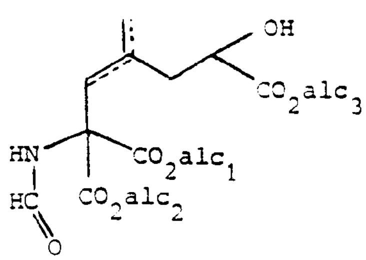

($II_A$)

dans laquelle $alc_1$, $alc_2$ et $alc_3$ identiques ou différents représentent un radical alkyle renfermant jusqu'à 8 atomes de carbone à l'action du chlorure de mésyle pour obtenir le composé de formule ($IV_A$) :

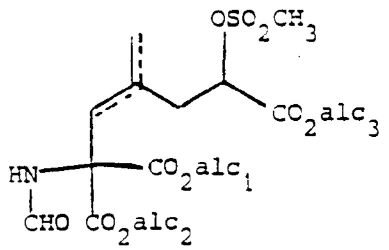

$(IV_A)$

que l'on soumet à l'action d'un agent de cyclisation pour obtenir le composé de formule $(I'_A)$ :

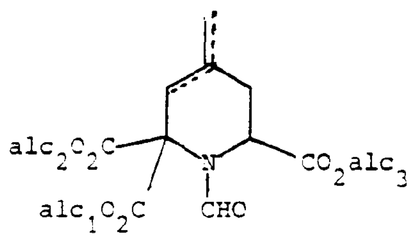

$(I'_A)$

que l'on soumet à l'action d'un agent de décarboxylation pour obtenir le composé de formule $(I''_A)$ :

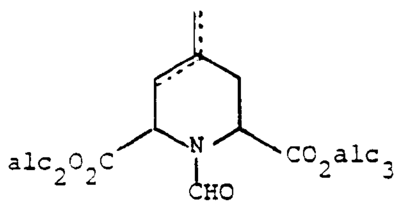

$(I''_A)$

3. Procédé selon la revendication 1, caractérisé en ce que l'on soumet un composé de formule $(II_B)$ :

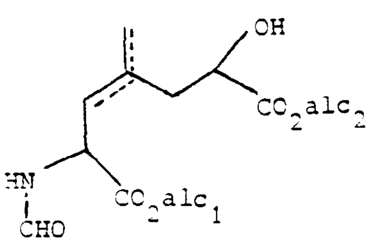

$(II_B)$

dans laquelle $alc_1$ et $alc_2$ identiques ou différents représentent un radical alkyle renfermant jusqu'à 8 atomes de carbone, à l'action d'un agent d'oxydation pour obtenir le composé de formule $(I_C)$ :

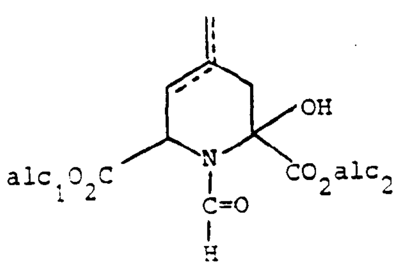

$(I'_C)$

4. Procédé de préparation selon la revendication 1, caractérisé en ce que l'on soumet un composé de formule $(II_C)$ :

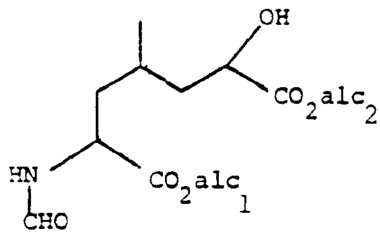

$(II_C)$

dans laquelle $alc_1$ et $alc_2$ identiques ou différents représentent un radical alkyle renfermant jusqu'à 8 atomes de carbone à l'action d'un agent de déprotection de la fonction amine, pour obtenir le composé de formule $(II_{B'})$ :

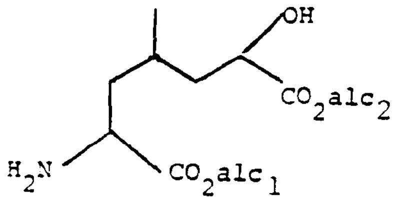

$(II_{B'})$

que l'on soumet à l'action du N-chlorosuccinimide ou du benzènesulfonate de 4-formyl 1-méthyl pyridinium, puis à l'action d'une base, puis à l'action d'un agent d'hydrolyse pour obtenir le composé de formule $(I'_B)$ :

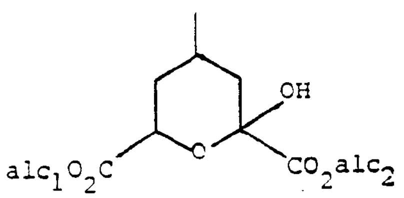

$(I'_B)$

5. Procédé selon la revendication 1, 2 ou 3, caractérisé en ce que l'on utilise au départ un composé de formule (II), $(II_A)$ ou $(II_B)$ dans laquelle les traits pointillés représentent une double liaison exo.

6. Procédé selon la revendication 1, 2 ou 3, caractérisé en ce que l'on utilise au départ un composé de formule (II), $(II_A)$ ou $(II_B)$ dans laquelle les traits pointillés ne représentent pas une double liaison.

7. Procédé selon la revendication 1, caractérisé en ce que l'on utilise au départ un composé de formule (II) dans laquelle $R_1$ et $R_2$ représentent un atome d'hydrogène.

8. Procédé selon la revendication 1, caractérisé en ce que l'on utilise au départ un composé de formule (II) dans laquelle Y' représente un radical hydroxyl libre ou protégé.

9. Procédé selon la revendication 1, caractérisé en ce que l'on utilise au départ un composé de formule (II) dans laquelle Y' représente un radical acétylénique protégé par un radical triméthylsilyl ou tout groupement protecteur connu de l'homme de métier que l'on soumet à l'action du fluorure de potassium ou de tétrabutylammonium ou tout autre agent de déprotection connu de l'homme de métier.

10. Procédé selon la revendication 1, caractérisé en ce que l'on utilise au départ un composé de formule (II) dans laquelle R représente un radical $CO_2alc$ dans lequel alc représente un radical alkyle renfermant jusqu'à 8 atomes de carbone et en ce que, le cas échéant, l'on hydrolyse pour obtenir un produit de formule (I) dans laquelle R' représente un atome d'hydrogène.

11. Procédé selon la revendication 10, caractérisé en ce que R représente un radical $CO_2\text{-}CH_3$.

**Revendications pour l'Etat contractant suivant : GR**

1. Procédé pour préparer les composés de formule (I) :

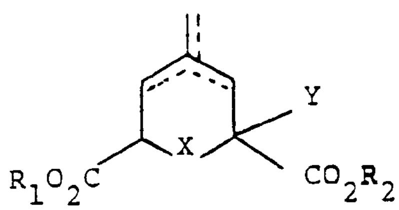

(I)

dans laquelle :

- les traits pointillés représentent une double liaison éventuelle endo ou exo ;
- $R_1$ et $R_2$ identiques ou différents représentent un atome d'hydrogène, un radical alkyle, alkényle, ou alkynyle renfermant jusqu'à 8 atomes de carbone,
  un radical aryle renfermant jusqu'à 14 atomes de carbone, un radical arylalkyle renfermant jusqu'à 18 atomes de carbone ou un radical

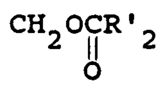

dans lequel $R'_2$ représente un radical alkyle renfermant jusqu'à 8 atomes de carbone ou un radical aryle renfermant jusqu'à 14 atomes de carbone ;

- X représente un atome d'oxygène ou un radical NR, R représentant un atome d'hydrogène, un radical CHO, un radical $CO_2R'$, R' représentant un atome d'hydrogène ou un radical alkyle renfermant jusqu'à 8 atomes de carbone ;
- Y représente un atome d'hydrogène, un radical alkyle, alkényle ou alkynyle renfermant jusqu'à 8 atomes de carbone éventuellement substitué par un ou plusieurs atomes d'halogène ou un radical hydroxyle, à la condition que si Y représente un radical hydroxyle, X ne représente pas un radical NH ainsi que leurs sels d'addition avec les acides organiques ou minéraux ou avec les bases, caractérisé en ce que l'on soumet un composé de formule (II) :

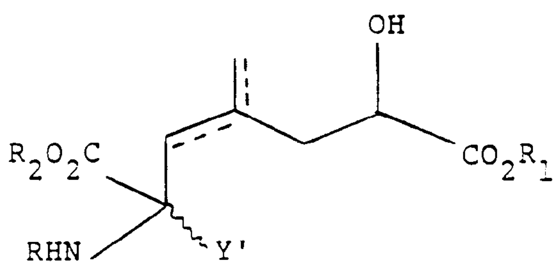

(II)

dans laquelle $R_1$ et $R_2$ conservent la même signification que précédemment, Y' représente soit les mêmes valeurs que Y, soit un précurseur de Y et R représente un radical CHO ou $CO_2$alc, alc représentant un radical alkyyle renfermant jusqu'à 8 atomes de carbone ;

ou bien à l'action d'un composé de formule (III) :

Hal $SO_2$alc (III)

dans laquelle Hal représente un atome d'halogène et alc représente un radical alkyle renfermant jusqu'à 8 atomes de carbone pour obtenir le composé de formule (IV) :

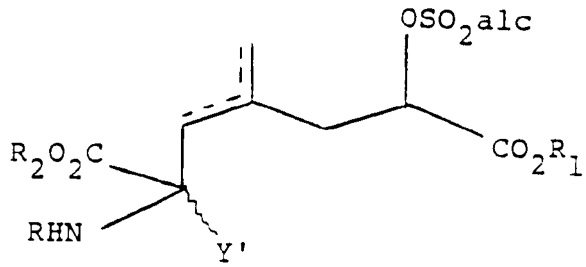

(IV)

que l'on soumet à l'action d'un agent favorisant la substitution nucléophile intramoléculaire pour obtenir le composé de formule ($I_A$) :

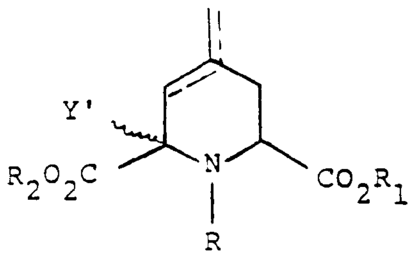

($I_A$)

ou bien dans le cas où Y' représente un atome d'hydrogène et R un radical CHO, à l'action d'un agent capable de libérer la fonction aminé pour obtenir le composé de formule (V) :

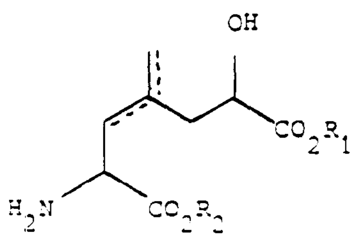

(V)

que l'on soumet à l'action du N-chlorosuccinimide ou du benzènesulfonate de 4-formyl 1-méthyl pyridinium, puis soumet le produit obtenu à l'action d'une base, puis soumet le produit obtenu à l'action d'un agent d'hydrolyse pour obtenir le composé de formule ($I_B$) :

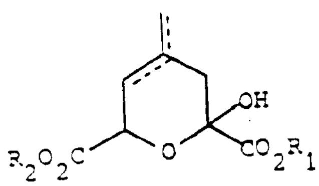

($I_B$)

ou bien dans le cas où Y représente un atome d'hydrogène et R un radical CHO à l'action d'un agent oxydant pour obtenir le composé de formule ($I_C$) :

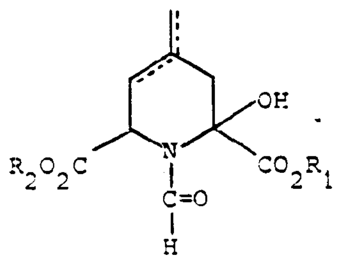

$(I_C)$

puis soumet le cas échéant les composés de formule $(I_A)$, $(I_B)$ et $(I_C)$ à la totalité ou à une partie seulement des opérations suivantes dans un ordre quelconque :

- réduction éventuelle de la double liaison endo ou exo ;
- traitement de Y' pour obtenir Y ;
- réduction totale ou sélective du substituant Y lorsque celui-ci est insaturé ;
- déprotection de la fonction aminé ;
- salification.

**2.** Procédé selon la revendication 1, caractérisé en ce que l'on soumet un composé de formule $(II_A)$ :

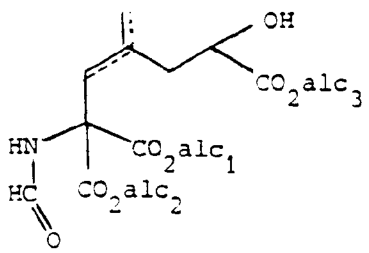

$(II_A)$

dans laquelle $alc_1$, $alc_2$ et $alc_3$ identiques ou différents représentent un radical alkyle renfermant jusqu'à 8 atomes de carbone à l'action du chlorure de mésyle pour obtenir le composé de formule $(IV_A)$ :

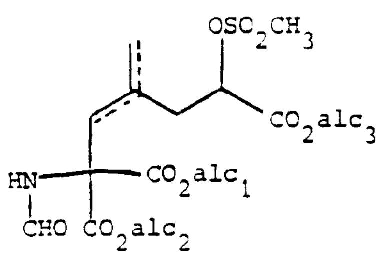

$(IV_A)$

que l'on soumet à l'action d'un agent de cyclisation pour obtenir le composé de formule $(I'_A)$ :

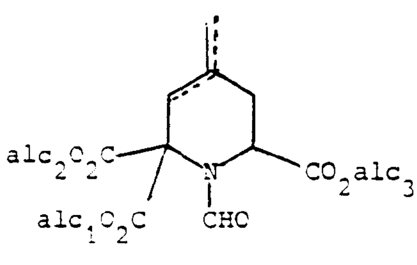

$(I'_A)$

que l'on soumet à l'action d'un agent de décarboxylation pour obtenir le composé de formule $(I''_A)$ :

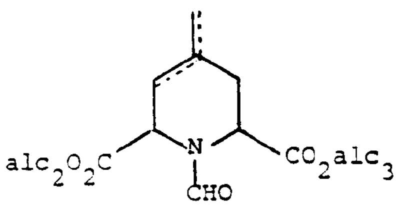

(I''$_A$)

3. Procédé selon la revendication 1, caractérisé en ce que l'on soumet un composé de formule ($II_B$) :

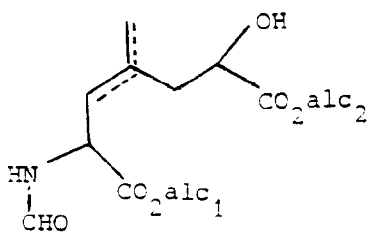

($II_B$)

dans laquelle $alc_1$ et $alc_2$ identiques ou différents représentent un radical alkyle renfermant jusqu'à 8 atomes de carbone, à l'action d'un agent d'oxydation pour obtenir le composé de formule ($I_{'C}$) :

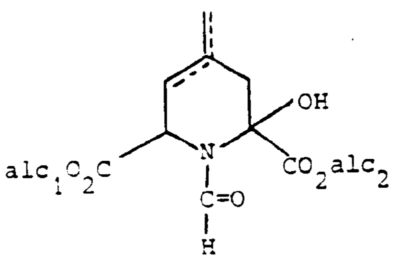

($I'_C$)

4. Procédé de préparation selon la revendication 1, caractérisé en ce que l'on soumet un composé de formule ($II_C$) :

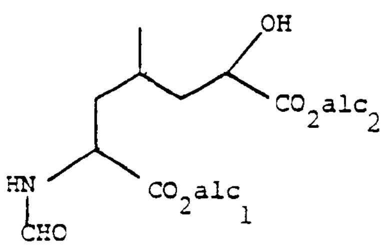

($II_C$)

dans laquelle $alc_1$ et $alc_2$ identiques ou différents représentent un radical alkyle renfermant jusqu'à 8 atomes de carbone à l'action d'un agent de déprotection de la fonction amine, pour obtenir le composé de formule ($II_{B'}$) :

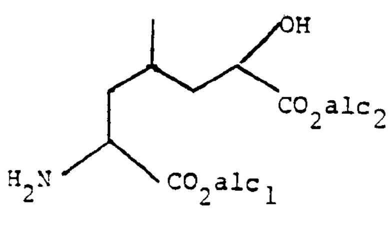

($II_{B'}$)

que l'on soumet à l'action du N-chlorosuccinimide ou du benzènesulfonate de 4-formyl 1-méthyl pyridinium, puis à l'action d'une base, puis à l'action d'un agent d'hydrolyse pour obtenir le composé de formule ($I'_B$) :

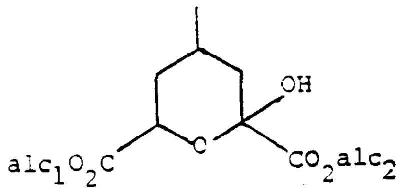

($I'_B$)

5. Procédé selon la revendication 1, 2 ou 3, caractérisé en ce que l'on utilise au départ un composé de formule (II), ($II_A$) ou ($II_B$) dans laquelle les traits pointillés représentent une double liaison exo.

6. Procédé selon la revendication 1, 2 ou 3, caractérisé en ce que l'on utilise au départ un composé de formule (II), ($II_A$) ou ($II_B$) dans laquelle les traits pointillés ne représentent pas une double liaison.

7. Procédé selon la revendication 1, caractérisé en ce que l'on utilise au départ un composé de formule (II) dans laquelle $R_1$ et $R_2$ représentent un atome d'hydrogène.

8. Procédé selon la revendication 1, caractérisé en ce que l'on utilise au départ un composé de formule (II) dans laquelle Y' représente un radical hydroxyl libre ou protégé.

9. Procédé selon la revendication 1, caractérisé en ce que l'on utilise au départ un composé de formule (II) dans laquelle Y' représente un radical acétylénique protégé par un radical triméthylsilyl ou tout groupement protecteur connu de l'homme de métier que l'on soumet à l'action du fluorure de potassium ou de tétrabutylammonium ou tout autre agent de déprotection connu de l'homme de métier.

10. Procédé selon la revendication 1, caractérisé en ce que l'on utilise au départ un composé de formule (II) dans laquelle R représente un radical $CO_2$alc dans lequel alc représente un radical alkyle renfermant jusqu'à 8 atomes de carbone et en ce que, le cas échéant, l'on hydrolyse pour obtenir un produit de formule (5) dans laquelle R' représente un atome d'hydrogène.

11. Procédé selon la revendication 10, caractérisé en ce que R représente un radical $CO_2$-$CH_3$.

12. Procédé selon la revendication 1, caractérisé en ce que l'on prépare l'un quelconque des produits dont les noms suivent :
    - l'acide 2-hydroxy-4-méthyl tétrahydro-2H-pyran-2,6-dicarboxylique ;
    - le 2-éthynyl-4-méthylène 1,2,6-pipéridine tricarboxylate de 1-méthyle (isomère A et B) ainsi que leurs sels d'addition avec les acides organiques ou minéraux ou avec les bases.

13. Procédé de préparation de compositions pharmaceutiques caractérisé en ce que l'on met à titre de principe actif l'un au moins des produits de formule (I) ou l'un au moins de leurs sels pharmaceutiquement acceptables tels que définis à la revendication 1 sous une forme destinée à cet usage.

14. Procédé selon la revendication 13, caractérisé en ce que le principe actif est choisi dans le groupe constitué par les produits nommés à la revendication 12 et leurs sels pharmaceutiquement acceptables.

## Patentansprüche

### Patentansprüche für folgende Vertragsstaaten : AT, BE, CH, DE, DK, FR, GB, IT, LI, LU, NL, SE

1. Verbindungen der Formel (I):

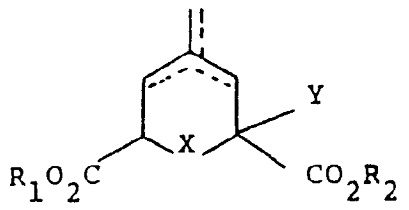

(I)

in der

- die punktierten Linien eine Doppelbindung darstellen, gegebenenfalls endo oder exo,
- $R_1$ und $R_2$, gleich oder verschieden, ein Wasserstoffatom, einen Alkyl-, Alkenyl- oder Alkinylrest mit bis zu 8 Kohlenstoffatomen, einen Arylrest mit bis zu 14 Kohlenstoffatomen, einen Arylalkylrest mit bis zu 18 Kohlenstoffatomen oder einen Rest

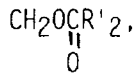

worin $R'_2$ einen Alkylrest mit bis zu 8 Kohlenstoffatomen darstellt, oder einen Arylrest mit bis zu 14 Kohlenstoffatomen bedeutet;
- X ein Sauerstoffatom oder ein Rest NR ist, R ein Wasserstoffatom, einen Rest CHO oder einen Rest $CO_2R'$ darstellt und R' ein Wasserstoffatom oder einen Alkylrest mit bis zu 8 Kohlenstoffatomen bedeutet;
- Y ein Wasserstoffatom oder einen Alkyl-, Alkenyl- oder Alkinylrest mit bis zu 8 Kohlenstoffatomen darstellt, gegebenenfalls substituiert durch ein oder mehrere Halogenatome oder Hydroxylreste, unter der Bedingung, daß, wenn Y ein Hydroxylrest ist, X keinen Rest NH bedeutet, sowie ihre Additionssalze mit organischen Säuren oder Mineralsäuren oder mit Basen.

**2.** Verbindungen nach Anspruch 1, in denen die punktierten Linien eine Doppelbindung exo darstellen, sowie ihre Additionssalze mit organischen Säuren oder Mineralsäuren oder mit Basen.

**3.** Verbindungen nach Anspruch 1, in denen die punktierten Linien keine Doppelbindung darstellen, sowie ihre Additionssalze mit organischen Säuren oder Mineralsäuren oder mit Basen.

**4.** Verbindungen nach Anspruch 1, 2 oder 3, in denen $R_1$ und $R_2$ ein Wasserstoffatom darstellen, sowie ihre Additionssalze mit organischen Säuren oder Mineralsäuren oder mit Basen.

**5.** Verbindungen nach einem der Ansprüche 1 bis 4, in denen Y einen Hydroxylrest darstellt, sowie ihre Additionssalze mit organischen Säuren oder Mineralsäuren oder mit Basen.

**6.** Verbindungen nach einem der Ansprüche 1 bis 4, in denen Y einen acetylenischen Rest darstellt, sowie ihre Additionssalze mit organischen Säuren oder Mineralsäuren oder mit Basen.

**7.** Verbindungen nach einem der Ansprüche 1 bis 6, in denen X ein Sauerstoffatom darstellt, sowie ihre Additionssalze mit organischen Säuren oder Mineralsäuren oder mit Basen.

**8.** Verbindungen nach einem der Ansprüche 1 bis 6, in denen X eine Gruppe $NCO_2R'$ darstellt und R' wie in Anspruch 1 definiert ist, sowie ihre Additionssalze mit organischen Säuren oder Mineralsäuren oder mit Basen.

**9.** Verbindungen nach Anspruch 7, in denen X einen Rest $NCO_2CH_3$ darstellt, sowie ihre Additionssalze mit organischen Säuren oder Mineralsäuren oder mit Basen.

**10.** Verbindungen wie in Anspruch 1 definiert, mit den folgenden Namen:
- 2-Hydroxy-4-methyl-tetrahydro-2H-pyran-2,6-dicarbonsäure;
- 2-Ethinyl-4-methylen-1,2,6-piperidin-tricarbonsäure-1-methylester (Isomer A und B), sowie ihre Additionssalze mit organischen Säuren oder Mineralsäuren oder mit Basen.

**11.** Als Medikamente die Verbindungen der Formel (I), wie in einem der Ansprüche 1 bis 9 definiert, sowie ihre Additionssalze mit pharmazeutisch akzeptablen organischen Säuren oder Mineralsäuren oder mit Basen.

**12.** Als Medikamente die Verbindungen der Formel (I), wie in Anspruch 10 definiert, sowie ihre Additionssalze mit pharmazeutisch akzeptablen organischen Säuren oder Mineralsäuren oder mit Basen.

**13.** Pharmazeutische Zusammensetzungen, die als Wirkstoff mindestens ein wie in Anspruch 11 oder 12 definiertes Medikament umfassen.

**14.** Verfahren zur Herstellung der Verbindungen der Formel (I) wie in Anspruch 1 definiert, dadurch gekennzeichnet, daß man eine Verbindung der Formel (II)

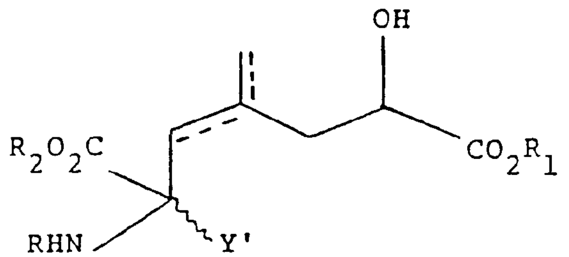

, (II)

in der $R_1$ und $R_2$ die gleiche Bedeutung wie vorstehend besitzen, Y' entweder die gleichen Werte wie Y aufweist oder ein Vorläufer von Y ist, und R einen Rest CHO oder $CO_2$alc bedeutet, worin alc einen Alkylrest mit bis zu 8 Kohlenstoffatomen darstellt;
entweder der Einwirkung einer Verbindung der Formel (III)

Hal $SO_2$alc (III)

unterzieht, in der Hal ein Halogenatom darstellt und alc einen Alkylrest mit bis zu 8 Kohlenstoffatomen bedeutet, um eine Verbindung der Formel (IV)

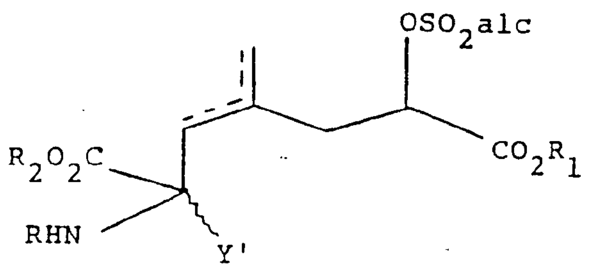

(IV)

zu erhalten, die man der Einwirkung eines die intramolekulare nucleophile Substitution begünstigenden Mittels unterzieht, um eine Verbindung der Formel ($I_A$)

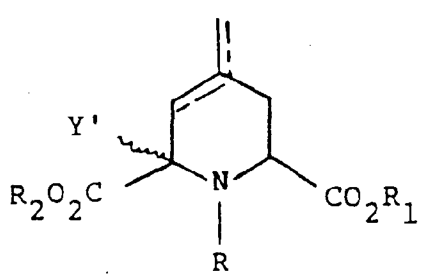

($I_A$)

zu erhalten,
oder in dem Fall, wo Y' ein Wasserstoffatom darstellt und R ein Rest CHO ist, der Einwirkung eines Mittels unterzieht, das fähig ist, die Aminfunktion freizusetzen, um die Verbindung der Formel (V)

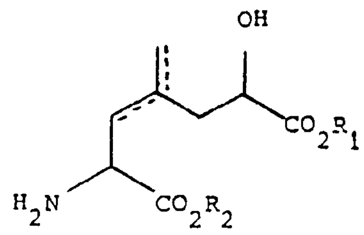

(V)

zu erhalten, die man der Einwirkung von N-Chlorsuccinimid oder 4-Formyl-1-methyl-pyridinium-benzolsulfonat unterzieht, man anschließend das erhaltene Produkt mit einer Base umsetzt und danach der Einwirkung eines Hydrolysierungsmittels unterzieht, um die Verbindung der Formel ($I_B$)

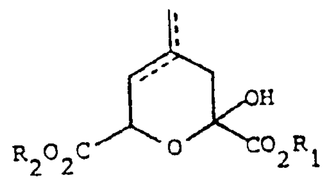

($I_B$)

zu erhalten,
<u>oder</u> in dem Fall, wo Y ein Wasserstoffatom darstellt und R ein Rest CHO ist, der Einwirkung eines Oxidationsmittels unterzieht, um die Verbindung der Formel ($I_C$)

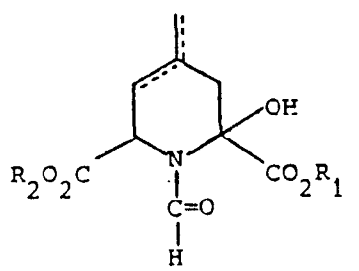

($I_C$)

zu erhalten, wonach man gegebenenfalls die Verbindungen der Formel ($I_A$), ($I_B$) und ($I_C$) vollständig oder nur teilweise den folgenden Operationen in beliebiger Reihenfolge unterzieht:

- gegebenenfalls Reduktion der Doppelbindung endo oder exo;
- Behandlung von Y', um Y zu erhalten;
- vollständige oder selektive Reduktion des Substituenten Y, wenn er ungesättigt ist;
- Entfernung der Schutzgruppe von der Aminfunktion;
- Salzbildung.

**15.** Verfahren nach Anspruch 14, dadurch gekennzeichnet, daß man eine Verbindung der Formel ($II_A$)

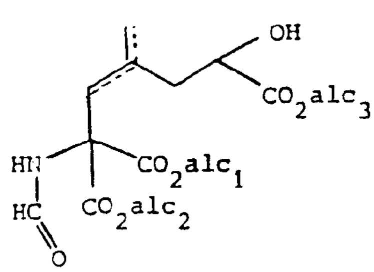

($II_A$)

in der $alc_1$, $alc_2$ und $alc_3$, gleich oder verschieden, einen Alkylrest mit bis zu 8 Kohlenstoffatomen darstellen, der Einwirkung von Mesylchlorid unterzieht, um die Verbindung der Formel ($IV_A$)

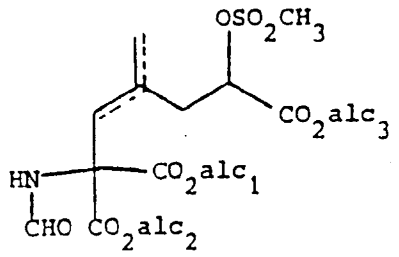

$(IV_A)$

zu erhalten, die man der Einwirkung eines Cyclisierungsmittels unterzieht, um die Verbindung der Formel $(I'_A)$

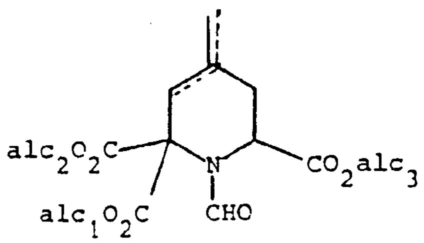

$(I'_A)$

zu erhalten, die man der Einwirkung eines Decarboxylierungsmittels unterzieht, um die Verbindung der Formel $(I''_A)$

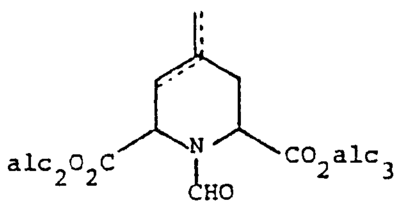

$(I''_A)$

zu erhalten.

**16.** Verfahren nach Anspruch 14, dadurch gekennzeichnet, daß man eine Verbindung der Formel $(II_B)$

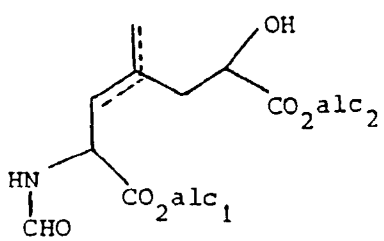

$(II_B)$

in der $alc_1$ und $alc_2$, gleich oder verschieden, einen Alkylrest mit bis zu 8 Kohlenstoffatomen darstellen, der Einwirkung eines Oxidationsmittels unterzieht, um die Verbindung der Formel $(I'_C)$

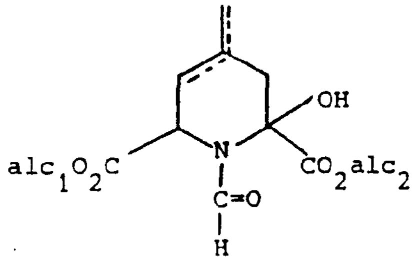

($I'_C$)

zu erhalten.

**17.** Verfahren zur Herstellung nach Anspruch 14, dadurch gekennzeichnet, daß man eine Verbindung der Formel ($II_C$)

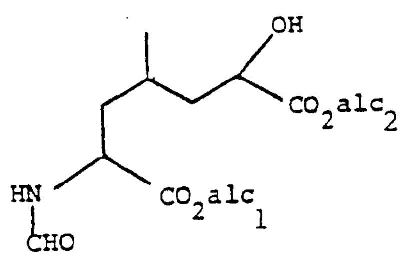

($II_C$)

in der $alc_1$ und $alc_2$, gleich oder verschieden, einen Alkylrest mit bis zu 8 Kohlenstoffatomen darstellen, der Einwirkung eines Mittels zur Entfernung der Schutzgruppe von der Aminfunktion unterzieht, um die Verbindung der Formel ($II_{B'}$)

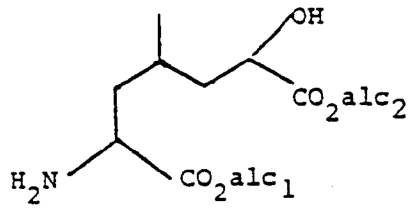

($II_{B'}$)

zu erhalten, die man der Einwirkung von N-Chlorsuccinimid oder 4-Formyl-1-methyl-pyridinium-benzolsulfonat unterzieht, man anschließend das erhaltene Produkt mit einer Base umsetzt und danach der Einwirkung eines Hydrolysierungsmittels unterzieht, um die Verbindung der Formel ($I'_B$)

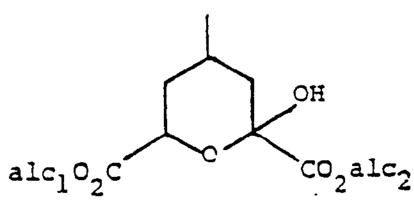

($I'_B$)

zu erhalten.

**Patentansprüche für folgenden Vertragsstaat : ES**

**1.** Verfahren zur Herstellung von Verbindungen der Formel (I):

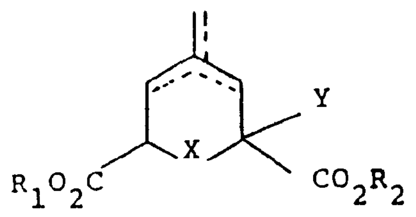

(I)

in der

- die punktierten Linien eine Doppelbindung darstellen, gegebenenfalls endo oder exo,
- $R_1$ und $R_2$, gleich oder verschieden, ein Wasserstoffatom, einen Alkyl-, Alkenyl- oder Alkinylrest mit bis zu 8 Kohlenstoffatomen, einen Arylrest mit bis zu 14 Kohlenstoffatomen, einen Arylalkylrest mit bis zu 18 Kohlenstoffatomen oder einen Rest

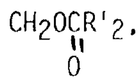

worin $R'_2$ einen Alkylrest mit bis zu 8 Kohlenstoffatomen darstellt, oder einen Arylrest mit bis zu 14 Kohlenstoffatomen bedeutet;

- X ein Sauerstoffatom oder ein Rest NR ist, R ein Wasserstoffatom, einen Rest CHO oder einen Rest $CO_2R'$ darstellt und R' ein Wasserstoffatom oder einen Alkylrest mit bis zu 8 Kohlenstoffatomen bedeutet;
- Y ein Wasserstoffatom oder einen Alkyl-, Alkenyl- oder Alkinylrest mit bis zu 8 Kohlenstoffatomen darstellt, gegebenenfalls substituiert durch ein oder mehrere Halogenatome oder Hydroxylreste, unter der Bedingung, daß, wenn Y ein Hydroxylrest ist, X keinen Rest NH bedeutet, sowie ihren Additionssalzen mit organischen Säuren oder Mineralsäuren oder mit Basen, dadurch gekennzeichnet, daß man eine Verbindung der Formel (II)

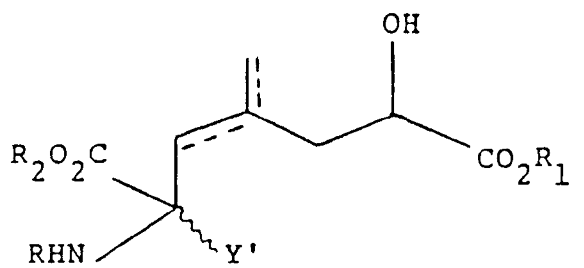

(II)

in der $R_1$ und $R_2$ die gleiche Bedeutung wie vorstehend besitzen, Y' entweder die gleichen Werte wie Y aufweist oder ein Vorläufer von Y ist, und R einen Rest CHO oder $CO_2$alc bedeutet, worin alc einen Alkylrest mit bis zu 8 Kohlenstoffatomen darstellt;

entweder der Einwirkung einer Verbindung der Formel (III)

Hal $SO_2$alc (III)

unterzieht, in der Hal ein Halogenatom darstellt und alc einen Alkylrest mit bis zu 8 Kohlenstoffatomen bedeutet, um eine Verbindung der Formel (IV)

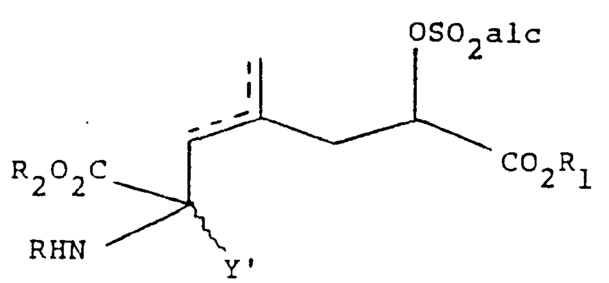

(IV)

zu erhalten, die man der Einwirkung eines die intramolekulare nucleophile Substitution begünstigenden Mittels unterzieht, um eine Verbindung der Formel ($I_A$)

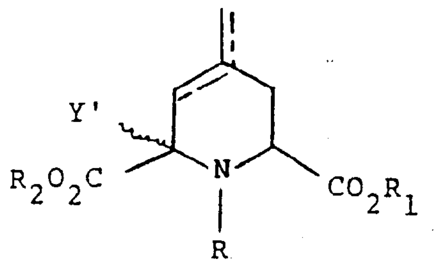

($I_A$)

zu erhalten,
oder in dem Fall, wo Y' ein Wasserstoffatom darstellt und R ein Rest CHO ist, der Einwirkung eines Mittels unterzieht, das fähig ist, die Aminfunktion freizusetzen, um die Verbindung der Formel (V)

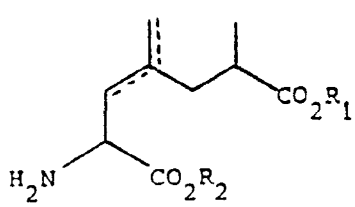

(V)

zu erhalten, die man der Einwirkung von N-Chlorsuccinimid oder 4-Formyl-1-methyl-pyridinium-benzolsulfonat unterzieht, man anschließend das erhaltene Produkt mit einer Base umsetzt und danach der Einwirkung eines Hydrolysierungsmittels unterzieht, um die Verbindung der Formel ($I_B$)

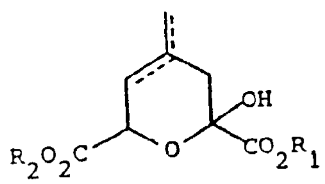

($I_B$)

zu erhalten,
oder in dem Fall, wo Y ein Wasserstoffatom darstellt und R ein Rest CHO ist, der Einwirkung eines Oxidationsmittels unterzieht, um die Verbindung der Formel ($I_C$)

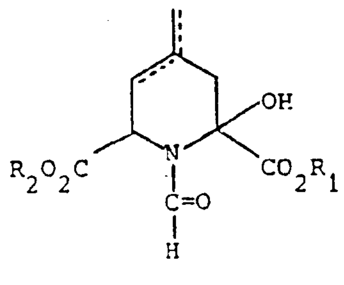

($I_C$)

zu erhalten, wonach man gegebenenfalls die Verbindungen der Formel ($I_A$), ($I_B$) und ($I_C$) vollständig oder nur teilweise den folgenden Operationen in beliebiger Reihenfolge unterzieht:
- gegebenenfalls Reduktion der Doppelbindung endo oder exo;
- Behandlung von Y', um Y zu erhalten;
- vollständige oder selektive Reduktion des Substituenten Y, wenn er ungesättigt ist;
- Entfernung der Schutzgruppe von der Aminfunktion;
- Salzbildung.

2. Verfahren nach Anspruch 1, dadurch gekennzeichnet, daß man eine Verbindung der Formel ($II_A$)

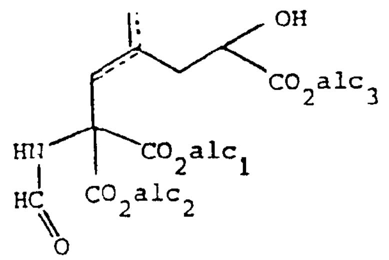

($II_A$)

in der $alc_1$, $alc_2$ und $alc_3$, gleich oder verschieden, einen Alkylrest mit bis zu 8 Kohlenstoffatomen darstellen, der Einwirkung von Mesylchlorid unterzieht, um die Verbindung der Formel ($IV_A$)

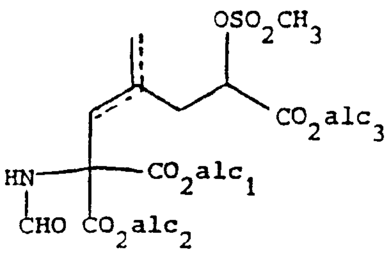

($IV_A$)

zu erhalten. die man der Einwirkung eines Cyclisierungsmittels unterzieht, um die Verbindung der Formel ($I'_A$)

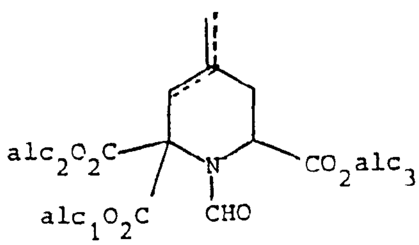

($I'_A$)

zu erhalten, die man der Einwirkung eines Decarboxylierungsmittels unterzieht, um die Verbindung der Formel ($I''_A$)

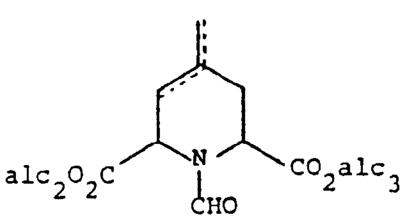

($I''_A$)

zu erhalten.

3. Verfahren nach Anspruch 1, dadurch gekennzeichnet, daß man eine Verbindung der Formel ($II_B$)

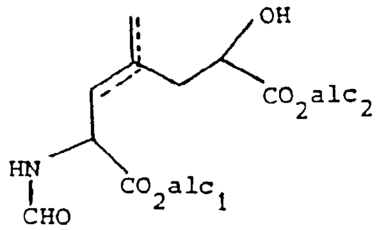

$(II_B)$

in der $alc_1$ und $alc_2$, gleich oder verschieden, einen Alkylrest mit bis zu 8 Kohlenstoffatomen darstellen, der Einwirkung eines Oxidationsmittels unterzieht, um die Verbindung der Formel $(I'_C)$

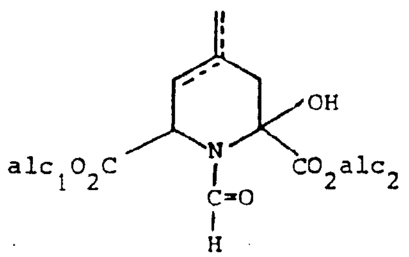

$(I'_C)$

zu erhalten.

4. Verfahren zur Herstellung nach Anspruch 1, dadurch gekennzeichnet, daß man eine Verbindung der Formel $(II_C)$

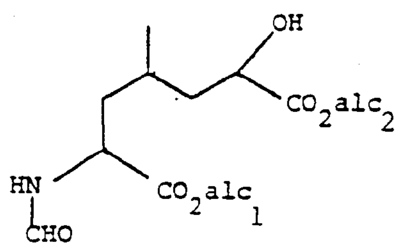

$(II_C)$

in der $alc_1$ und $alc_2$, gleich oder verschieden, einen Alkylrest mit bis zu 8 Kohlenstoffatomen darstellen, der Einwirkung eines Mittels zur Entfernung der Schutzgruppe von der Aminfunktion unterzieht, um die Verbindung der Formel $(II_{B'})$

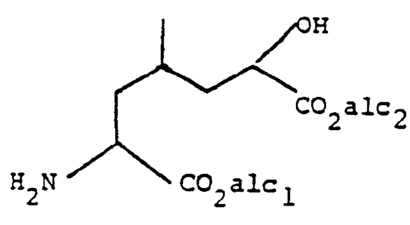

$(II_{B'})$

zu erhalten, die man der Einwirkung von N-Chlorsuccinimid oder 4-Formyl-1-methyl-pyridinium-benzolsulfonat unterzieht, man anschließend das erhaltene Produkt mit einer Base umsetzt und danach der Einwirkung eines Hydrolysierungsmittels unterzieht, um die Verbindung der Formel $(I'_B)$

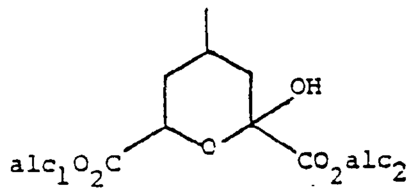

$(I'_B)$

zu erhalten.

5. Verfahren nach Anspruch 1, 2 oder 3, dadurch gekennzeichnet, daß man als Ausgangsprodukt eine Verbindung der Formel (II), $(II_A)$ oder $(II_B)$ verwendet, in der die punktierten Linien eine Doppelbindung exo darstellen.

6. Verfahren nach Anspruch 1, 2 oder 3, dadurch gekennzeichnet, daß man als Ausgangsprodukt eine Verbindung der Formel (II), $(II_A)$ oder $(II_B)$ verwendet, in der die punktierten Linien keine Doppelbindung darstellen.

7. Verfahren nach Anspruch 1, dadurch gekennzeichnet, daß man als Ausgangsprodukt eine Verbindung der Formel (II) verwendet, in der $R_1$ und $R_2$ ein Wasserstoffatom darstellen.

8. Verfahren nach Anspruch 1, dadurch gekennzeichnet, daß man als Ausgangsprodukt eine Verbindung der Formel (II) verwendet, in der Y' einen freien oder geschützten Hydroxylrest darstellt.

9. Verfahren nach Anspruch 1, dadurch gekennzeichnet, daß man als Ausgangsprodukt eine Verbindung der Formel (II) verwendet, in der Y' einen acetylenischen Rest darstellt, geschützt durch einen Trimethylsilylrest oder jede andere dem Fachmann bekannte Schutzgruppe, wobei man die genannte Verbindung der Einwirkung von Kaliumfluorid oder Tetrabutylammoniumfluorid unterzieht, oder jedem anderen dem Fachmann bekannten Mittel zur Entfernung der Schutzgruppe.

10. Verfahren nach Anspruch 1, dadurch gekennzeichnet, daß man als Ausgangsprodukt eine Verbindung der Formel (II) verwendet, in der R einen Rest $CO_2$alc darstellt, worin alc einen Alkylrest mit bis zu 8 Kohlenstoffatomen bedeutet, und dadurch, daß man gegebenenfalls hydrolysiert, um ein Produkt der Formel (I) zu erhalten, in der R' ein Wasserstoffatom ist.

11. Verfahren nach Anspruch 10, dadurch gekennzeichnet, daß R einen Rest $CO_2$-$CH_3$ darstellt.

**Patentansprüche für folgenden Vertragsstaat : GR**

1. Verfahren zur Herstellung von Verbindungen der Formel (I):

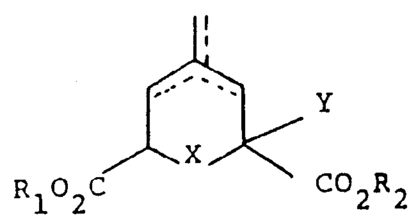

(I)

in der
- die punktierten Linien eine Doppelbindung darstellen, gegebenenfalls endo oder exo,
- $R_1$ und $R_2$, gleich oder verschieden, ein Wasserstoffatom, einen Alkyl-, Alkenyl- oder Alkinylrest mit bis zu 8 Kohlenstoffatomen, einen Arylrest mit bis zu 14 Kohlenstoffatomen, einen Arylalkylrest mit bis zu 18 Kohlenstoffatomen oder einen Rest

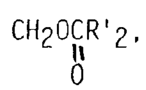

worin $R'_2$ einen Alkylrest mit bis zu 8 Kohlenstoffatomen darstellt, oder einen Arylrest mit bis zu 14 Kohlenstoffatomen bedeutet;

- X ein Sauerstoffatom oder ein Rest NR ist, R ein Wasserstoffatom, einen Rest CHO oder einen Rest $CO_2R'$ darstellt und R' ein Wasserstoffatom oder einen Alkylrest mit bis zu 8 Kohlenstoffatomen bedeutet;
- Y ein Wasserstoffatom oder einen Alkyl-, Alkenyl- oder Alkinylrest mit bis zu 8 Kohlenstoffatomen darstellt, gegebenenfalls substituiert durch ein oder mehrere Halogenatome oder Hydroxylreste, unter der Bedingung, daß, wenn Y ein Hydroxylrest ist, X keinen Rest NH bedeutet, sowie ihren Additionssalzen mit organischen Säuren oder Mineralsäuren oder mit Basen, dadurch gekennzeichnet, daß man eine Verbindung der Formel (II)

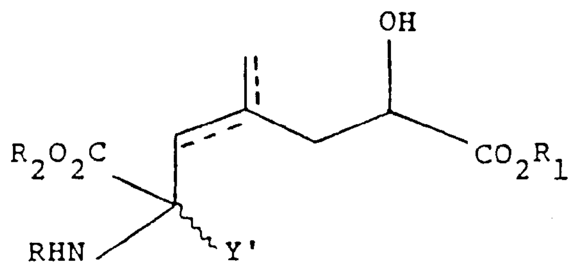

(II)

in der $R_1$ und $R_2$ die gleiche Bedeutung wie vorstehend besitzen, Y' entweder die gleichen Werte wie Y aufweist oder ein Vorläufer von Y ist, und R einen Rest CHO oder $CO_2$alc bedeutet, worin alc einen Alkylrest mit bis zu 8 Kohlenstoffatomen darstellt;
<u>entweder</u> der Einwirkung einer Verbindung der Formel (III)

Hal $SO_2$alc (III)

unterzieht, in der Hal ein Halogenatom darstellt und alc einen Alkylrest mit bis zu 8 Kohlenstoffatomen bedeutet, um eine Verbindung der Formel (IV)

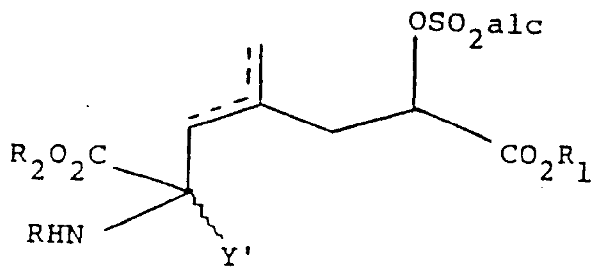

(IV)

zu erhalten, die man der Einwirkung eines die intramolekulare nucleophile Substitution begünstigenden Mittels unterzieht, um eine Verbindung der Formel ($I_A$)

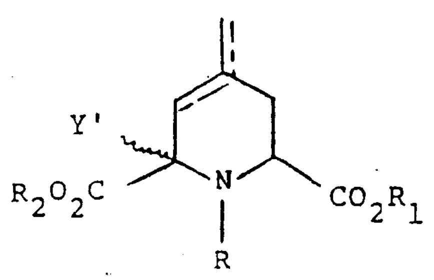

($I_A$)

zu erhalten,
<u>oder</u> in dem Fall, wo Y' ein Wasserstoffatom darstellt und R ein Rest CHO ist, der Einwirkung eines Mittels unterzieht, das fähig ist, die Aminfunktion freizusetzen, um die Verbindung der Formel (V)

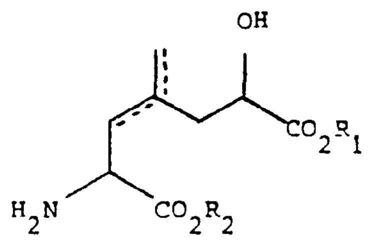

(V)

zu erhalten, die man der Einwirkung von N-Chlorsuccinimid oder 4-Formyl-1-methyl-pyridinium-benzolsulfonat unterzieht, man anschließend das erhaltene Produkt mit einer Base umsetzt und danach der Einwirkung eines Hydrolysierungsmittels unterzieht, um die Verbindung der Formel ($I_B$)

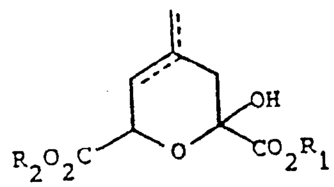

($I_B$)

zu erhalten,
oder in dem Fall, wo Y ein Wasserstoffatom darstellt und R ein Rest CHO ist, der Einwirkung eines Oxidationsmittels unterzieht, um die Verbindung der Formel ($I_C$)

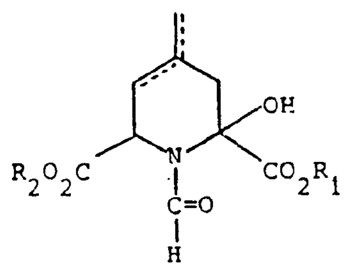

($I_C$)

zu erhalten, wonach man gegebenenfalls die Verbindungen der Formel ($I_A$), ($I_B$) und ($I_C$) vollständig oder nur teilweise den folgenden Operationen in beliebiger Reihenfolge unterzieht:

- gegebenenfalls Reduktion der Doppelbindung endo oder exo;
- Behandlung von Y', um Y zu erhalten;
- vollständige oder selektive Reduktion des Substituenten Y, wenn er ungesättigt ist;
- Entfernung der Schutzgruppe von der Aminfunktion;
- Salzbildung.

**2.** Verfahren nach Anspruch 1, dadurch gekennzeichnet, daß man eine Verbindung der Formel ($II_A$)

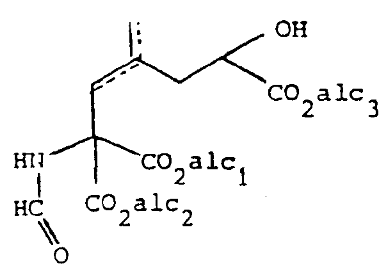

($II_A$)

in der $alc_1$, $alc_2$ und $alc_3$, gleich oder verschieden, einen Alkylrest mit bis zu 8 Kohlenstoffatomen darstellen, der Einwirkung von Mesylchlorid unterzieht, um die Verbindung der Formel ($IV_A$)

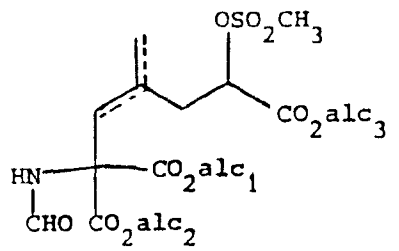

$(IV_A)$

zu erhalten, die man der Einwirkung eines Cyclisierungsmittels unterzieht, um die Verbindung der Formel $(I'_A)$

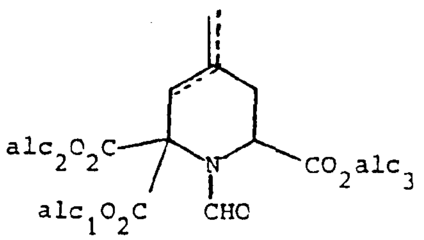

$(I'_A)$

zu erhalten, die man der Einwirkung eines Decarboxylierungsmittels unterzieht, um die Verbindung der Formel $(I''_A)$

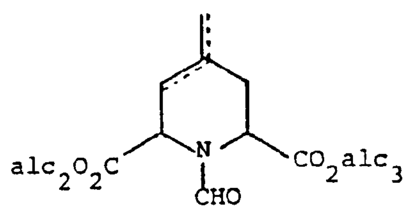

$(I''_A)$

zu erhalten.

3. Verfahren nach Anspruch 1, dadurch gekennzeichnet, daß man eine Verbindung der Formel $(II_B)$

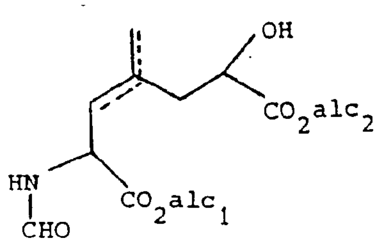

$(II_B)$

in der $alc_1$ und $alc_2$, gleich oder verschieden, einen Alkylrest mit bis zu 8 Kohlenstoffatomen darstellen, der Einwirkung eines Oxidationsmittels unterzieht, um die Verbindung der Formel $(I'_C)$

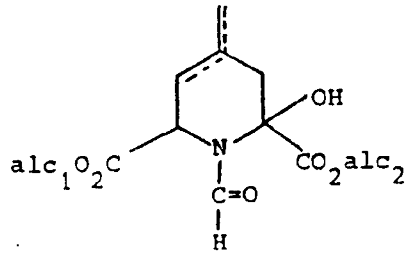

($I'_C$)

zu erhalten.

4. Verfahren zur Herstellung nach Anspruch 1, dadurch gekennzeichnet, daß man eine Verbindung der Formel ($II_C$)

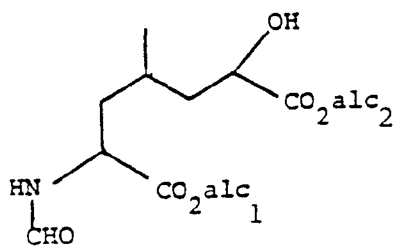

($II_C$)

in der $alc_1$ und $alc_2$, gleich oder verschieden, einen Alkylrest mit bis zu 8 Kohlenstoffatomen darstellen, der Einwirkung eines Mittels zur Entfernung der Schutzgruppe von der Aminfunktion unterzieht, um die Verbindung der Formel ($II_B$')

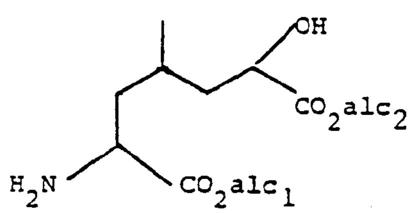

($II_{B'}$)

zu erhalten, die man der Einwirkung von N-Chlorsuccinimid oder 4-Formyl-1-methyl-pyridinium-benzolsulfonat unterzieht, man anschließend das erhaltene Produkt mit einer Base umsetzt und danach der Einwirkung eines Hydrolysierungsmittels unterzieht, um die Verbindung der Formel ($I'_B$)

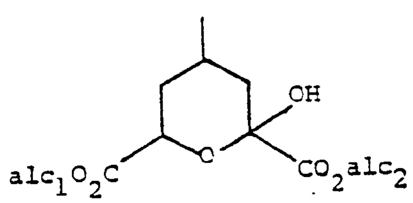

($I'_B$)

zu erhalten.

5. Verfahren nach Anspruch 1, 2 oder 3, dadurch gekennzeichnet, daß man als Ausgangsprodukt eine Verbindung der Formel (II), ($II_A$) oder ($II_B$) verwendet, in der die punktierten Linien eine Doppelbindung exo darstellen.

6. Verfahren nach Anspruch 1, 2 oder 3, dadurch gekennzeichnet, daß man als Ausgangsprodukt eine Verbindung der Formel (II), ($II_A$) oder ($II_B$) verwendet, in der die punktierten Linien keine Doppelbindung darstellen.

7. Verfahren nach Anspruch 1, dadurch gekennzeichnet, daß man als Ausgangsprodukt eine Verbindung der Formel (II) verwendet, in der $R_1$ und $R_2$ ein Wasserstoffatom darstellen.

8. Verfahren nach Anspruch 1, dadurch gekennzeichnet, daß man als Ausgangsprodukt eine Verbindung der Formel (II) verwendet, in der Y' einen freien oder geschützten Hydroxylrest darstellt.

9. Verfahren nach Anspruch 1, dadurch gekennzeichnet, daß man als Ausgangsprodukt eine Verbindung der Formel (II) verwendet, in der Y' einen acetylenischen Rest darstellt, geschützt durch einen Trimethylsilylrest oder jede andere dem Fachmann bekannte Schutzgruppe, wobei man die genannte Verbindung der Einwirkung von Kaliumfluorid oder Tetrabutylammoniumfluorid unterzieht, oder jedem anderen dem Fachmann bekannten Mittel zur Entfernung der Schutzgruppe.

10. Verfahren nach Anspruch 1, dadurch gekennzeichnet, daß man als Ausgangsprodukt eine Verbindung der Formel (II) verwendet, in der R einen Rest $CO_2$alc darstellt, worin alc einen Alkylrest mit bis zu 8 Kohlenstoffatomen bedeutet, und dadurch, daß man gegebenenfalls hydrolysiert, um ein Produkt der Formel (I) zu erhalten, in der R' ein Wasserstoffatom ist.

11. Verfahren nach Anspruch 10, dadurch gekennzeichnet, daß R einen Rest $CO_2$-$CH_3$ darstellt.

12. Verfahren nach Anspruch 1, dadurch gekennzeichnet, daß man irgendeines der Produkte mit den folgenden Namen herstellt:
   - 2-Hydroxy-4-methyl-tetrahydro-2H-pyran-2,6-dicarbonsäure;
   - 2-Ethinyl-4-methylen-1,2,6-piperidin-tricarbonsäure-1-methylester (Isomer A und B), sowie ihre Additionssalze mit organischen Säuren oder Mineralsäuren oder mit Basen.

13. Verfahren zur Herstellung von pharmazeutischen Zusammensetzungen, dadurch gekennzeichnet, daß man als Wirkstoff mindestens eines der Produkte der Formel (I) oder mindestens eines ihrer pharmazeutisch akzeptablen Salze, wie in Anspruch 1 definiert, in einer für diesen Gebrauch bestimmten Form verwendet,

14. Verfahren nach Anspruch 13, dadurch gekennzeichnet, daß der Wirkstoff aus der Gruppe ausgewählt wird, bestehend aus den in Anspruch 12 bezeichneten Produkten und ihren pharmazeutisch akzeptablen Salzen.

## Claims

**Claims for the following Contracting States : AT, BE, CH, DE, DK, FR, GB, IT, LI, LU, NL, SE**

1. The compounds of formula (I):

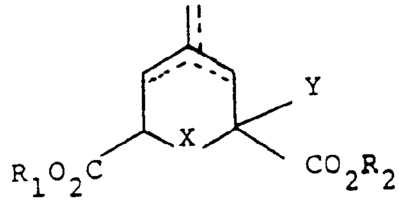

(I)

in which:
   - the dotted lines represent an optional endo or exo double bond;
   - $R_1$ and $R_2$, identical or different, represent a hydrogen atom, an alkyl, alkenyl or alkynyl radical containing up to 8 carbon atoms,
     an aryl radical containing up to 14 carbon atoms, an arylalkyl radical containing up to 18 carbon atoms or a

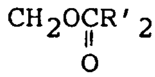

radical in which $R'_2$ represents an alkyl radical containing up to 8 carbon atoms or an aryl radical containing up to 14 carbon atoms;
- X represents an oxygen atom or an NR radical, R representing a hydrogen atom, a CHO radical, a $CO_2R'$ radical, R' representing a hydrogen atom or an alkyl radical containing up to 8 carbon atoms;
- Y represents a hydrogen atom, an alkyl, alkenyl or alkynyl radical containing up to 8 carbon atoms optionally substituted by one or more halogen atoms or a hydroxyl radical, on the condition that if Y represents a hydroxyl radical, X does not represent an NH radical, as well as their addition salts with organic or mineral acids or with bases.

**2.** The compounds according to claim 1 in which the dotted lines represent an exo double bond, as well as their addition salts with organic or mineral acids or with bases.

**3.** The compounds according to claim 1 in which the dotted lines do not represent a double bond as well as their addition salts with organic or mineral acids or with bases.

**4.** The compounds according to claim 1, 2 or 3 in which $R_1$ and $R_2$ represent a hydrogen atom as well as their addition salts with organic or mineral acids or with bases.

**5.** The compounds according to any one of claims 1 to 4 in which Y represents a hydroxyl radical as well as their addition salts with organic or mineral acids or with bases.

**6.** The compounds according to any one of claims 1 to 4 in which Y represents an acetylenic radical as well as their addition salts with organic or mineral acids or with bases.

**7.** The compounds according to any one of claims 1 to 6 in which X represents an oxygen atom as well as their addition salts with organic or mineral acids or with bases.

**8.** The compounds according to any one of claims 1 to 6 in which X represents an $NCO_2R'$ group, R' being defined as in claim 1, as well as their addition salts with organic or mineral acids or with bases.

**9.** The compounds according to claim 7 in which X represents an $NCO_2CH_3$ radical as well as their addition salts with organic acids or with bases.

**10.** The compounds defined in claim 1 the names of which follow:
- 2-hydroxy-4-methyl tetrahydro-2H-pyran-2,6-dicarboxylic acid;
- 1-methyl 2-ethynyl-4-methylene 1,2,6-piperidine tricarboxylate (A and B isomer) as well as their addition salts with organic or mineral acids or with bases.

**11.** As medicaments, the compounds of formula (I) defined in any one of claims 1 to 9 as well as their addition salts with pharmaceutically acceptable organic or mineral acids or with pharmaceutically acceptable bases.

**12.** As a medicament, the compounds of formula (I) defined in claim 10, as well as their addition salts with pharmaceutically acceptable organic or mineral acids or with pharmaceutically acceptable bases.

**13.** The pharmaceutical compositions containing at least one medicament defined in claim 11 or 12 as active ingredient.

**14.** Preparation process for the compounds of formula (I) as defined in claim 1, characterized in that a compound of formula (II):

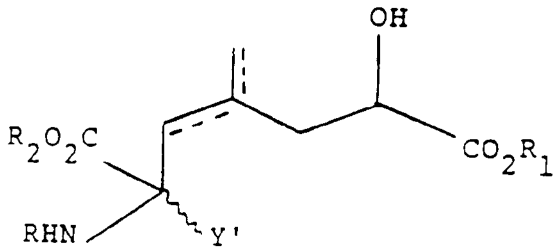

(II)

in which $R_1$ and $R_2$ retain the same meaning as previously, Y' represents either the same values as Y or a precursor of Y and R represents a CHO or $CO_2$alk radical, alk representing an alkyl radical containing up to 8 carbon atoms, is subjected either to the action of a compound of formula (III):

Hal $SO_2$alk (III)

in which Hal represents a halogen atom and alk represents an alkyl radical containing up to 8 carbon atoms, in order to obtain the compound of formula (IV):

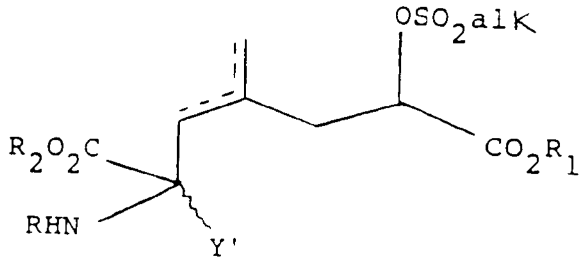

(IV)

which is subjected to the action of an agent which promotes intramolecular nucleophilic substitution in order to obtain the compound of formula ($I_A$):

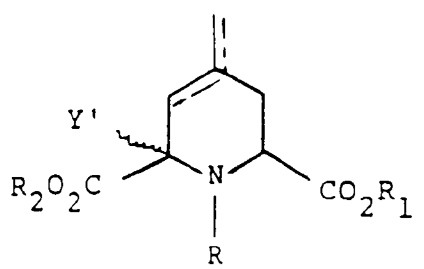

($I_A$)

or in the case where Y' represents a hydrogen atom and R represents a CHO radical, to the action of an agent capable of releasing the amine function in order to obtain the compound of formula (V):

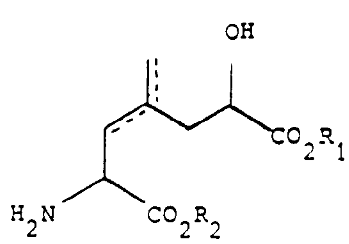

(V)

which is subjected to the action of N-chlorosuccinimide or 4-formyl 1-methyl pyridinium benzenesulphonate, then the product obtained is subjected to the action of a base, then the product obtained is subjected to the action of an hydrolysis agent in order to obtain the compound of formula ($I_B$):

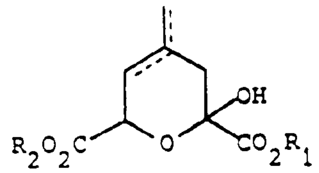

$(I_B)$

or in the case where Y represents a hydrogen atom and R represents a CHO radical to the action of an oxidising agent in order to obtain the compound of formula ($I_C$):

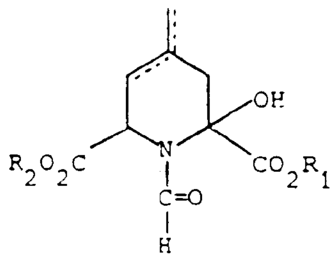

$(I_C)$

then, if appropriate, the compounds of formulae ($I_A$), ($I_B$) and ($I_C$) are subjected to all or only a part of the following operations, in any order:

- optional reduction of the endo or exo double bond;
- treatment of Y' in order to obtain Y;
- total or selective reduction of the Y substituent when this is unsaturated;
- deprotection of the amino function;
- salification.

**15.** Process according to claim 14, characterized in that a compound of formula ($II_A$):

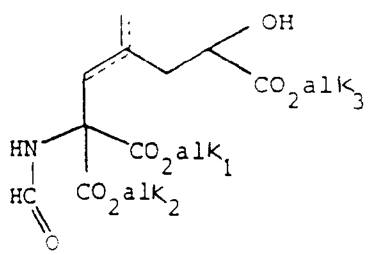

$(II_A)$

in which $alk_1$, $alk_2$ and $alk_3$, identical or different, represent an alkyl radical containing up to 8 carbon atoms, is subjected to the action of mesyl chloride in order to obtain the compound of formula ($IV_A$):

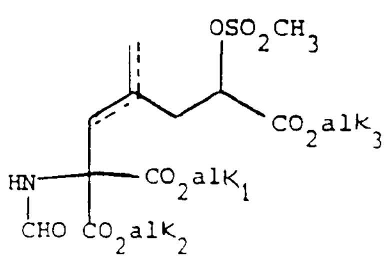

$(IV_A)$

which is subjected to the action of a cyclization agent in order to obtain the compound of formula ($I'_A$):

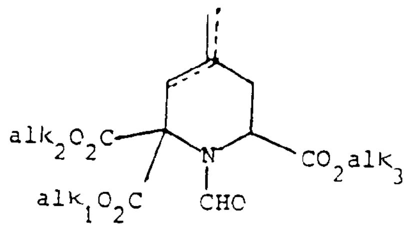

($I'_A$)

which is subjected to the action of a decarboxylation agent in order to obtain the compound of formula ($I''_A$):

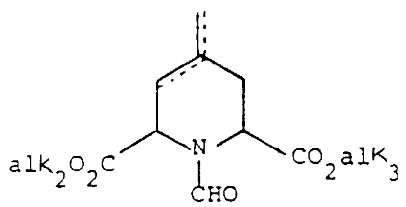

($I''_A$)

**16.** Process according to claim 14, characterized in that a compound of formula ($II_B$):

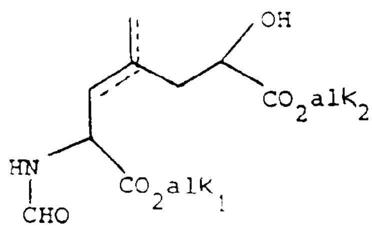

($II_B$)

in which $alk_1$ and $alk_2$, identical or different, represent an alkyl radical containing up to 8 carbon atoms, is subjected to the action of an oxidizing agent in order to obtain the compound of formula ($I'_C$):

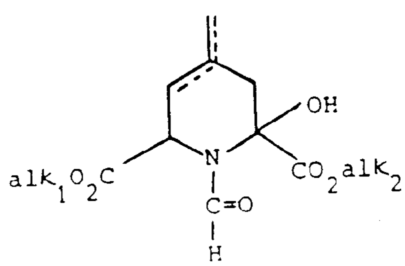

($I'_C$)

**17.** Preparation process according to claim 14, characterized in that a compound of formula ($II_C$):

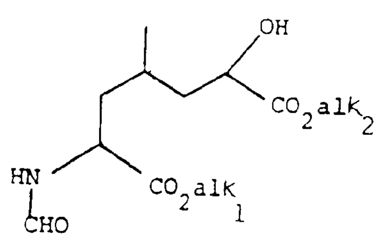

($II_C$)

in which $alk_1$ and $alk_2$, identical or different, represent an alkyl radical containing up to 8 carbon atoms, is subjected to the action of a deprotection agent of the amine function in order to obtain the compound of formula ($II_{B'}$):

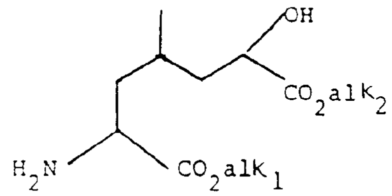

$(II_{B'})$

which is subjected to the action of N-chlorosuccinimide or of 4-formyl 1-methyl pyridinium benzenesulphonate, then to the action of a base, then to the action of a hydrolysis agent in order to obtain the compound of formula $(I'_B)$:

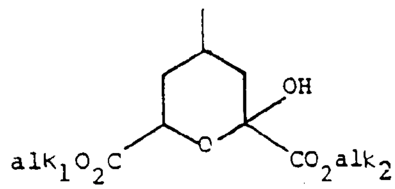

$(I'_B)$

**Claims for the following Contracting State : ES**

1. Process for preparing the compounds of formula (I):

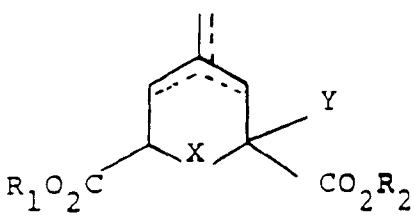

(I)

in which:
- the dotted lines represent an optional endo or exo double bond;
- $R_1$ and $R_2$, identical or different, represent a hydrogen atom, an alkyl, alkenyl or alkynyl radical containing up to 8 carbon atoms,
  an aryl radical containing up to 14 carbon atoms, an arylalkyl radical containing up to 18 carbon atoms or a

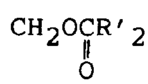

  radical in which $R'_2$ represents an alkyl radical containing up to 8 carbon atoms or an aryl radical containing up to 14 carbon atoms;
- X represents an oxygen atom or an NR radical, R representing a hydrogen atom, a CHO radical, a $CO_2R'$ radical, R' representing a hydrogen atom or an alkyl radical containing up to 8 carbon atoms;
- Y represents a hydrogen atom, an alkyl, alkenyl or alkynyl radical containing up to 8 carbon atoms optionally substituted by one or more halogen atoms or a hydroxyl radical, on the condition that if Y represents a hydroxyl radical, X does not represent an NH radical, as well as their addition salts with organic or mineral acids or with bases, characterized in that a compound of formula (II):

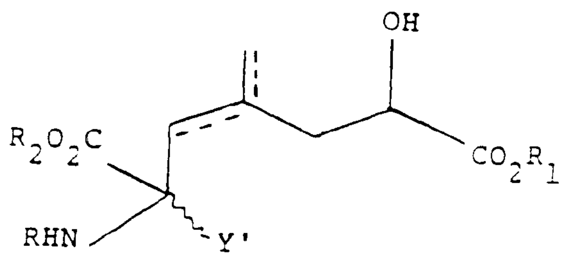

(II)

in which $R_1$ and $R_2$ retain the same meaning as previously, Y' represents either the same values as Y or a precursor of Y and R represents a CHO or $CO_2$alk radical, alk representing an alkyl radical containing up to 8 carbon atoms, is subjected either to the action of a compound of formula (III):

Hal $SO_2$alk (III)

in which Hal represents a halogen atom and alk represents an alkyl radical containing up to 8 carbon atoms, in order to obtain the compound of formula (IV):

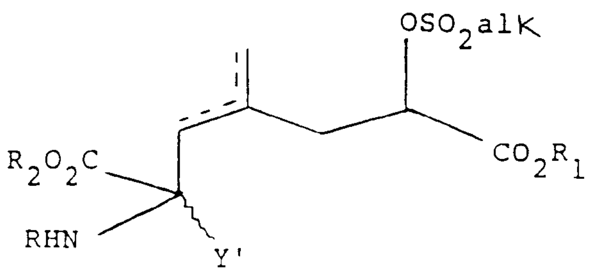

(IV)

which is subjected to the action of an agent which promotes intramolecular nucleophilic substitution in order to obtain the compound of formula ($I_A$):

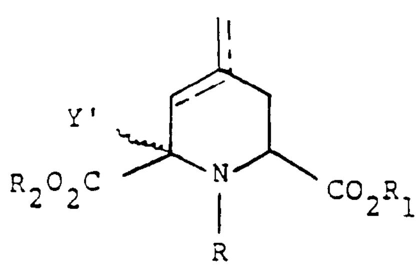

($I_A$)

or in the case where Y' represents a hydrogen atom and R represents a CHO radical, to the action of an agent capable of releasing the amine function in order to obtain the compound of formula (V):

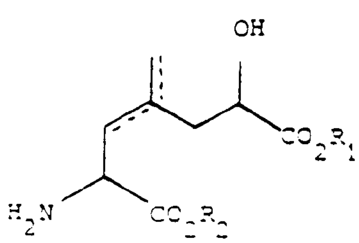

(V)

which is subjected to the action of N-chlorosuccinimide or 4-formyl 1-methyl pyridinium benzenesulphonate, then the product obtained is subjected to the action of a base, then the product obtained is subjected to the action of an hydrolysis agent in order to obtain the compound of formula ($I_B$):

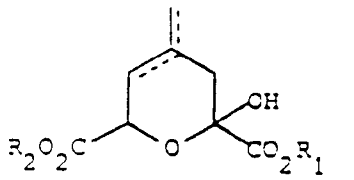

$(I_B)$

or in the case where Y represents a hydrogen atom and R represents a CHO radical, to the action of an oxidising agent in order to obtain the compound of formula ($I_C$):

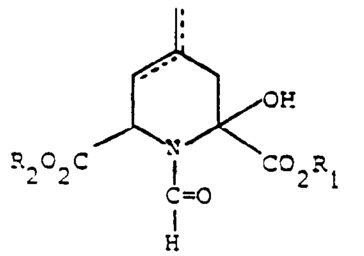

$(I_C)$

then, if appropriate, the compounds of formulae ($I_A$), ($I_B$) and ($I_C$) are subjected to all or only a part of the following operations, in any order:

- optional reduction of the endo or exo double bond;
- treatment of Y' in order to obtain Y;
- total or selective reduction of the Y substituent when this is unsaturated;
- deprotection of the amino function;
- salification.

**2.** Process according to claim 1, characterized in that a compound of formula ($II_A$):

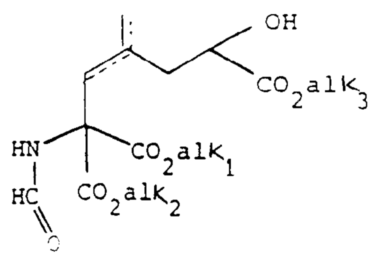

$(II_A)$

in which $alk_1$, $alk_2$ and $alk_3$, identical or different, represent an alkyl radical containing up to 8 carbon atoms, is subjected to the action of mesyl chloride in order to obtain the compound of formula ($IV_A$):

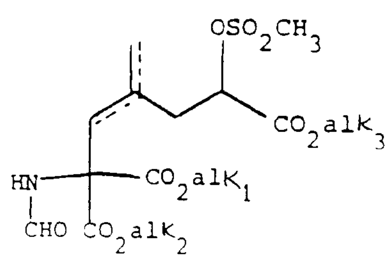

$(IV_A)$

which is subjected to the action of a cyclization agent in order to obtain the compound of formula ($I'_A$):

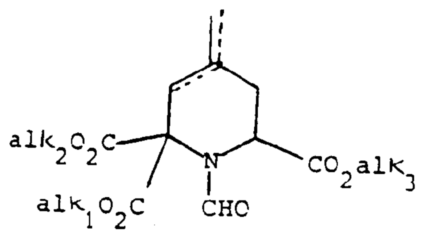

($I'_A$)

which is subjected to the action of a decarboxylation agent in order to obtain the compound of formula ($I''_A$):

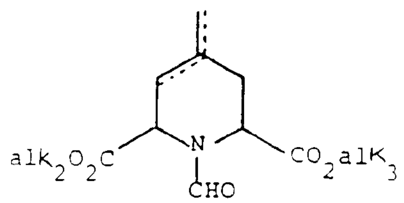

($I''_A$)

3. Process according to claim 1, characterized in that a compound of formula ($II_B$):

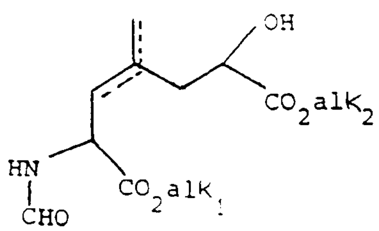

($II_B$)

in which $alk_1$ and $alk_2$, identical or different, represent an alkyl radical containing up to 8 carbon atoms, is subjected to the action of an oxidizing agent in order to obtain the compound of formula ($I'_C$):

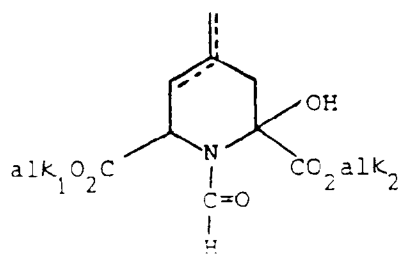

($I'_C$)

4. Preparation process according to claim 1, characterized in that a compound of formula ($II_C$):

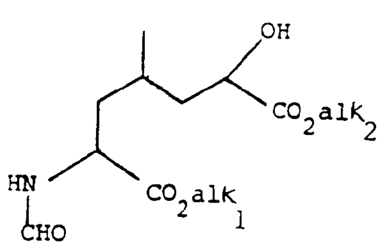

($II_C$)

in which $alk_1$ and $alk_2$, identical or different, represent an alkyl radical containing up to 8 carbon atoms is subjected to the action of a deprotection agent of the amine function in order to obtain the compound of formula ($II_{B'}$):

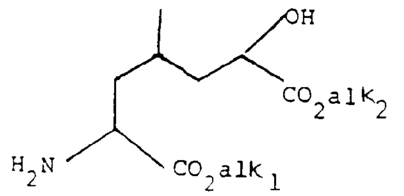

$(II_{B'})$

which is subjected to the action of N-chlorosuccinimide or of 4-formyl 1-methyl pyridinium benzenesulphonate, then to the action of a base, then to the action of a hydrolysis agent in order to obtain the compound of formula ($I'_B$):

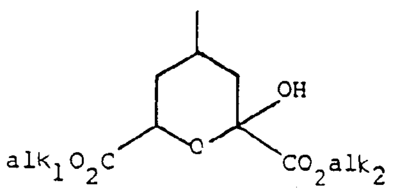

$(I'_B)$

5. Process according to claim 1, 2 or 3, characterized in that a compound of formula (II), ($II_A$) or ($II_B$) is used at the start in which the dotted lines represent an exo double bond.

6. Process according to claim 1, 2 or 3, characterized in that a compound of formula (II), ($II_A$) or ($II_B$) is used at the start in which the dotted lines do not represent a double bond.

7. Process according to claim 1, characterized in that a compound of formula (II) is used at the start in which $R_1$ and $R_2$ represent a hydrogen atom.

8. Process according to claim 1, characterized in that a compound of formula (II) is used at the start in which Y' represents a free or protected hydroxyl radical.

9. Process according to claim 1, characterized in that a compound of formula (II) is used at the start in which Y' represents an acetylenic group protected by a trimethylsilyl radical or any protective group known to an average man skilled in the art which is subjected to the action of potassium or tetrabutylammonium fluoride or any other deprotection agent known to an average man skilled in the art.

10. Process according to claim 1, characterized in that a compound of formula (II) is used at the start in which R is a $CO_2$alk radical in which alk represents an alkyl radical containing up to 8 carbon atoms and in that, if appropriate, it is hydrolyzed in order to obtain a product of formula (I) in which R' represents a hydrogen atom.

11. Process according to claim 10, characterized in that R represents a $CO_2$-$CH_3$ radical.

**Claims for the following Contracting State : GR**

1. Process for preparing the compounds of formula (I):

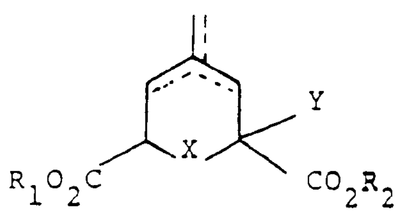

(I)

in which:
- the dotted lines represent an optional endo or exo double bond;
- $R_1$ and $R_2$, identical or different, represent a hydrogen atom, an alkyl, alkenyl or alkynyl radical containing up to 8 carbon atoms,
an aryl radical containing up to 14 carbon atoms, an arylalkyl radical containing up to 18 carbon atoms or a

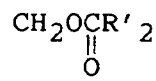

radical in which $R'_2$ represents an alkyl radical containing up to 8 carbon atoms or an aryl radical containing up to 14 carbon atoms;

- X represents an oxygen atom or an NR radical, R representing a hydrogen atom, a CHO radical, a $CO_2R'$ radical, R' representing a hydrogen atom or an alkyl radical containing up to 8 carbon atoms;
- Y represents a hydrogen atom, an alkyl, alkenyl or alkynyl radical containing up to 8 carbon atoms optionally substituted by one or more halogen atoms or a hydroxyl radical, on the condition that if Y represents a hydroxyl radical, X cannot represent an NH radical, as well as their addition salts with organic or mineral acids or with bases, characterized in that a compound of formula (II):

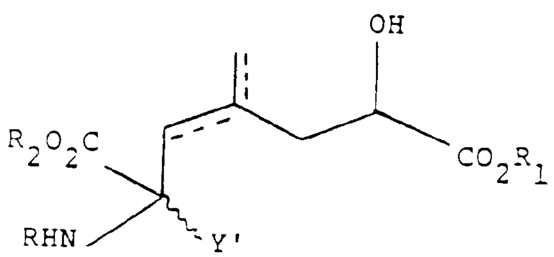

(II)

in which $R_1$ and $R_2$ retain the same meaning as previously, Y' represents either the same values as Y or a precursor of Y and R represents a CHO or $CO_2$alk radical, alk representing an alkyl radical containing up to 8 carbon atoms, is subjected either to the action of a compound of formula (III):

Hal $SO_2$alk (III)

in which Hal represents a halogen atom and alk represents an alkyl radical containing up to 8 carbon atoms, in order to obtain the compound of formula (IV):

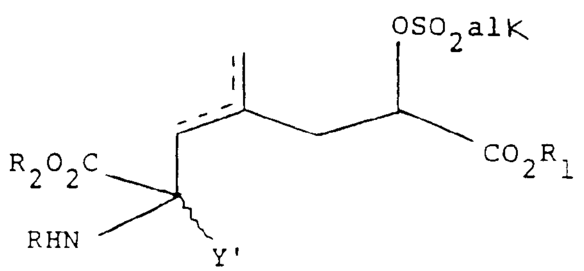

(IV)

which is subjected to the action of an agent which promotes intramolecular nucleophilic substitution in order to obtain the compound of formula ($I_A$):

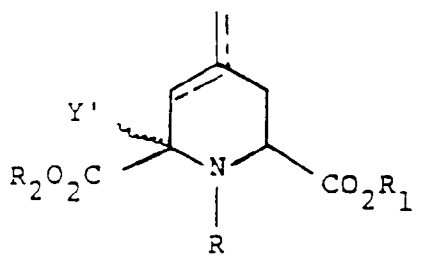

($I_A$)

or in the case where Y' represents a hydrogen atom and R represents a CHO radical, to the action

of an agent capable of releasing the amine function in order to obtain the compound of formula (V):

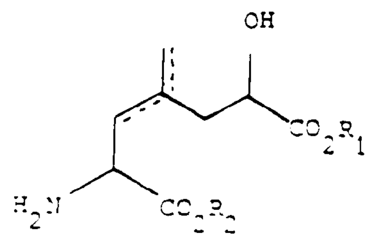

(V)

which is subjected to the action of N-chlorosuccinimide or 4-formyl 1-methyl pyridinium benzenesulphonate, then the product obtained is subjected to the action of a base, then the product obtained is subjected to the action of an hydrolysis agent in order to obtain the compound of formula ($I_B$):

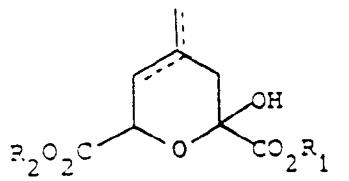

($I_B$)

or in the case where Y represents a hydrogen atom and R represents a CHO radical, to the action of an oxidising agent in order to obtain the compound of formula ($I_C$):

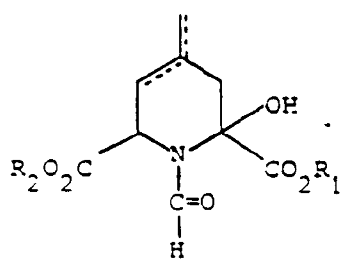

($I_C$)

then, if appropriate, the compounds of formulae ($I_A$), ($I_B$) and ($I_C$) are subjected to all or only a part of the following operations, in any order:

- optional reduction of the endo or exo double bond;
- treatment of Y' in order to obtain Y;
- total or selective reduction of the Y substituent when this is unsaturated;
- deprotection of the amino function;
- salification.

2. Process according to claim 1, characterized in that a compound of formula ($II_A$):

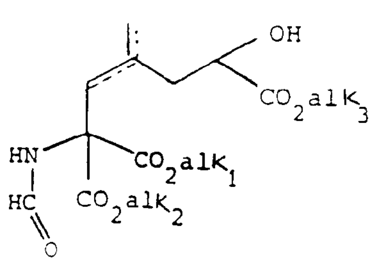

($II_A$)

in which $alk_1$, $alk_2$ and $alk_3$, identical or different, represent an alkyl radical containing up to 8 carbon atoms, is subjected to the action of mesyl chloride in order to obtain the compound of formula ($IV_A$):

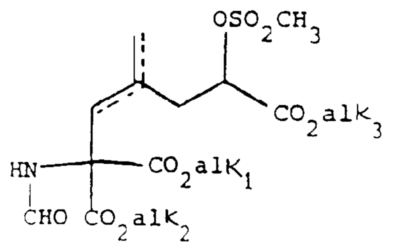

$(IV_A)$

which is subjected to the action of a cyclization agent in order to obtain the compound of formula ($I'_A$):

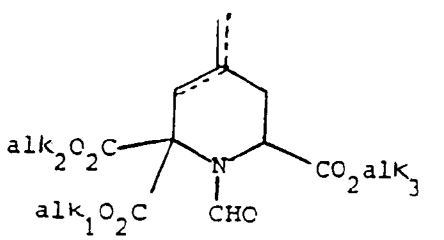

$(I'_A)$

which is subjected to the action of a decarboxylation agent in order to obtain the compound of formula ($I''_A$):

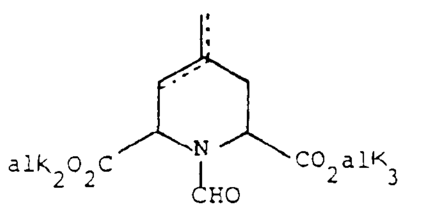

$(I''_A)$

**3.** Process according to claim 1, characterized in that a compound of formula ($II_B$):

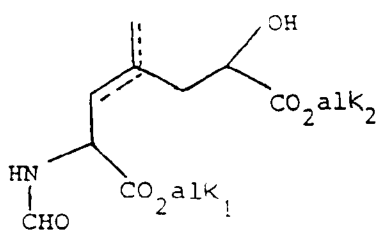

$(II_B)$

in which $alk_1$ and $alk_2$, identical or different, represent an alkyl radical containing up to 8 carbon atoms, is subjected to the action of an oxidizing agent in order to obtain the compound of formula ($I'_C$):

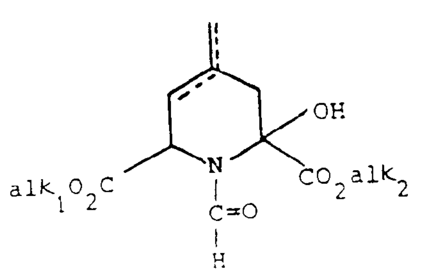

$(I'_C)$

**4.** Preparation process according to claim 1, characterized in that a compound of formula ($II_C$):

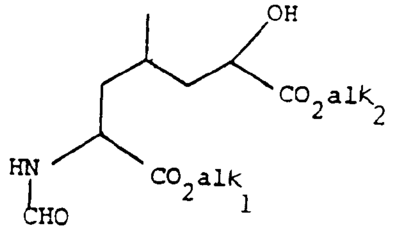

(IIC)

in which $alk_1$ and $alk_2$, identical or different, represent an alkyl radical containing up to 8 carbon atoms is subjected to the action of a deprotection agent of the amine function in order to obtain the compound of formula ($II_{B'}$):

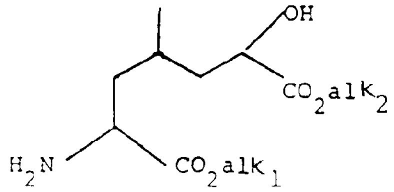

($II_{B'}$)

which is subjected to the action of N-chlorosuccinimide or of 4-formyl 1-methyl pyridinium benzenesulphonate, then to the action of a base, then to the action of a hydrolysis agent in order to obtain the compound of formula ($I'_B$):

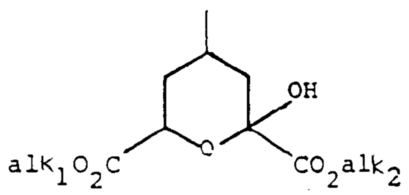

($I'_B$)

5. Process according to claim 1, 2 or 3, characterized in that a compound of formula (II), ($II_A$) or ($II_B$) is used at the start in which the dotted lines represent an exo double bond.

6. Process according to claim 1, 2 or 3, characterized in that a compound of formula (II), ($II_A$) or ($II_B$) is used at the start in which the dotted lines do not represent a double bond.

7. Process according to claim 1, characterized in that a compound of formula (II) is used at the start in which $R_1$ and $R_2$ represent a hydrogen atom.

8. Process according to claim 1, characterized in that a compound of formula (II) is used at the start in which Y' represents a free or protected hydroxyl radical.

9. Process according to claim 1, characterized in that a compound of formula (II) is used at the start in which Y' represents an acetylenic group protected by a trimethylsilyl radical or any protective group known to an average man skilled in the art which is subjected to the action of potassium or tetrabutylammonium fluoride or any other deprotection agent known to an average man skilled in the art.

10. Process according to claim 1, characterized in that a compound of formula (II) is used at the start in which R is a $CO_2$alk radical in which alk represents an alkyl radical containing up to 8 carbon atoms and in that, if appropriate, it is hydrolyzed in order to obtain a product of formula (I) in which R' represents a hydrogen atom.

11. Process according to claim 10, characterized in that R represents a $CO_2$-$CH_3$ radical.

12. Process according to claim 1, characterized in that any one of the products are prepared the names of which follow:

- 2-hydroxy-4-methyl tetrahydro-2H-pyran-2,6-dicarboxylic acid;
- 1-methyl 2-ethynyl-4-methylene 1,2,6-piperidine tricarboxylate (A and B isomer) as well as their addition salts with organic or mineral acids or with bases.

**13.** Preparation process for pharmaceutical compositions characterized in that at least one of the products of formula (I) or at least one of their pharmaceutically acceptable salts as defined in claim 1 are used as active ingredient in a form intended for this use.

**14.** Process according to claim 13, characterized in that the active ingredient is chosen from the group constituted by the products named in claim 12 and their pharmaceutically acceptable salts.